# EUROPÄISCHE PATENTANMELDUNG

(11) **EP 4 847 068 A1**
(43) Veröffentlichungstag der Anmeldung: **02.12.2026**
(21) Anmeldenummer: 26180159.1
(22) Anmeldetag: 21.05.2026
(51) Int. Cl.: A61M 16/00, F04D 29/66

(54) **BEATMUNGSGERÄT**

(30) Priorität: 27.05.2025 DE 102025120787
(71) Anmelder: Löwenstein Medical Technology S.A., 2557 Luxembourg (LU)
(72) Erfinder: Gerlach, Angela, 22549 Hamburg (DE); Veers, Kathleen, 24116 Kiel (DE); Oster, Alexander, 24214 Gettorf (DE); Maaß, Jan Christoph, 25451 Quickborn (DE)
(74) Vertreter: Löwenstein Medical IP

(57) **Zusammenfassung**

Die Erfindung betrifft ein Beatmungsgerät umfassend ein einen Innenraum des Beatmungsgerätes begrenzendes Gehäuse mit einem Atemgaseingang und einem Atemgasausgang, ein im Innenraum angeordnetes Gebläse, das ausgebildet ist, um Atemgas vom Atemgaseingang zum Atemgasausgang zu fördern, eine im Innenraum angeordnete Kammer, die eine Öffnung zum Atemgasaustausch mit dem Atemgaseingang und eine zweite Öffnung zum Atemgasaustausch mit dem Gebläse umfasst, wobei das Gebläse eingerichtet ist, um Atemgas über die zweite Öffnung aus der Kammer anzusaugen und zum Atemgasausgang zu fördern, wobei die Kammer eine ein Lumen der Kammer umschließende Kammerwand umfasst, die derart beabstandet vom Gehäuse angeordnet ist, dass ein Spalt zwischen der Kammerwand und dem Gehäuse ausgebildet ist, der verhindert, dass Schwingungen von der Kammerwand auf das Gehäuse übertragen werden.

## Beschreibung

### Technisches Gebiet

Die Erfindung betrifft ein Beatmungsgerät.

### Stand der Technik

Ein Beatmungsgerät ist eine Vorrichtung zur Beatmung von Individuen mit unzureichender, gestörter oder ausgesetzter Eigenatmung. Beatmungsgeräte können sowohl im häuslichen Bereich als auch im klinischen Bereich Anwendung finden. Beatmungsgeräte umfassen in der Regel einen Einlass, einen Auslass sowie einen Atemgasantrieb (Gebläse) zur Förderung eines Atemgases. Zwischen dem Einlass und dem Auslass bildet das Beatmungsgerät einen Atemgasweg, in dem das Gebläse angeordnet sein kann.

Bei Beatmungsgeräten bildet die Schallunterdrückung eine bedeutende Herausforderung. Die Betriebsgeräusche von Beatmungsgeräten entstehen durch Schallwellen, die beispielsweise vom Gebläse erzeugt werden. Zur Verminderung der Betriebsgeräusche wurden in der Vergangenheit häufig schalldämmende Schäume im oder am Atemgasweg eingesetzt. Diese können jedoch Schwierigkeiten bei einem automatisierten Zusammenbau des Beatmungsgerätes verursachen.

Der Erfindung liegt deshalb die Aufgabe zugrunde, ein leises und kompaktes Beatmungsgerät bereitzustellen, das einfach herzustellen ist und sicher betrieben werden kann.

Diese Aufgabe wird durch die Gegenstände der unabhängigen Ansprüche gelöst. Vorteilhafte Ausführungsformen der Erfindung sind in den abhängigen Ansprüchen, der nachfolgenden Beschreibung und den beigefügten Figuren dargelegt.

Die Erfindung betrifft ein Beatmungsgerät umfassend ein einen Innenraum des Beatmungsgerätes begrenzendes Gehäuse mit einem Atemgaseingang und einem Atemgasausgang. Das Beatmungsgerät umfasst zudem ein im Innenraum angeordnetes Gebläse, das ausgebildet ist, um das Atemgas vom Atemgaseingang zum Atemgasausgang zu fördern. Das Beatmungsgerät umfasst zudem eine im Innenraum angeordnete Kammer, die eine Öffnung zum Atemgasaustausch mit dem Atemgaseingang und eine zweite Öffnung zum Atemgasaustausch mit dem Gebläse umfasst. Das Gebläse ist eingerichtet, um Atemgas über die zweite Öffnung aus der Kammer anzusaugen und zum Atemgasausgang zu fördern. Die Kammer umfasst eine ein Lumen der Kammer umschließende Kammerwand, die derart beabstandet vom Gehäuse angeordnet ist, dass ein Spalt zwischen der Kammerwand und dem Gehäuse ausgebildet ist, der verhindert, dass Schwingungen von der Kammerwand (direkt) auf das Gehäuse übertragen werden.

Im Folgenden werden verschiedene Ausführungsformen der Erfindung beschrieben. Diese Ausführungsformen sind nicht als Beschränkung des Umfangs der Erfindung zu verstehen.

Unter "Atemgas" kann ein einzelnes Atemgas oder ein Gemisch aus mehreren Atemgasen verstanden werden. Unter Atemgas kann beispielsweise normale Atemluft aus der Umgebung, Sauerstoff, Stickstoff, Kohlendioxid, Wasserdampf, Narkosegas oder mit einer dieser Stoffe angereicherte Atemluft aus der Umgebung verstanden werden.

Unter "Beatmungsgerät" kann eine Vorrichtung zur Beatmung und/oder zur Narkotisierung verstanden werden. Das Beatmungsgerät kann somit eine Beatmungsfunktion und eine Anästhesiefunktion und eine Kombination aus diesen aufweisen. Unter "Beatmung" kann eine künstliche Beatmung, eine Atemunterstützung, eine Atemtherapie, eine Atemdiagnose, eine Hustenunterstützung, eine Sauerstoffliefernde Therapie wie beispielweise eine High-Flow-Therapie, eine Inhalationsnarkose und eine Kombination aus mindestens zwei dieser Beispiele verstanden werden. Somit kann ein Individuum unter Verwendung des Beatmungsgerätes beatmet oder in der Atmung unterstützt werden und alternativ oder zusätzlich auch unter Narkose gehalten werden. Zu diesem Zwecke kann das Beatmungsgerät auch mit Anästhesiegasen betrieben werden und somit einer Inhalationsanästhesie dienen.

Unter einem Beatmungsgerät können somit sämtliche Geräte verstanden werden, die ein Individuum bei der natürlichen Atmung unterstützen und/oder die Beatmung übernehmen und/oder einer Atemtherapie dienen und/oder einer Inhalationsnarkose dienen und/oder anderweitig auf die Atmung des Patienten oder Anwenders einwirken. Ein Beatmungsgerät kann zum Beispiel ein Beatmungsgerät für klinische oder Heimanwendungen, ein Atemtherapiegerät, ein CPAP-, APAP- oder BiLevel-Gerät, ein Highflow-Therapiegerät, ein Narkose- bzw. Anästhesiegerät, ein Notfall-Beatmungsgerät, ein Sauerstofflieferndes Gerät, ein Diagnosesystem oder ein Hustentherapiegerät bzw. eine Hustenmaschine sein.

Das Gehäuse des Beatmungsgerätes ist ausgebildet, um das Innere des Beatmungsgerätes atemgasdicht gegenüber der Umgebungsluft abzugrenzen und einen Atemgasaustausch nur über definierte Ein- und Ausgänge zuzulassen. Der Atemgaseingang und der Atemgasausgang des Beatmungsgerätes können am Gehäuse angeordnet sein und/oder durch das Gehäuse ausgebildet sein. Der Atemgaseinlass ist ausgebildet, um Atemgas in das Innere des Beatmungsgerätes hineinzulassen. Der Atemgasauslass ist ausgebildet, um Atemgas aus dem Inneren des Beatmungsgerätes herauszulassen. Das Beatmungsgerät kann auch mehrere Eingänge aufweisen, über die alternativ oder zusätzlich weitere Atemgase in den Atemgasweg eingeleitet werden können wie beispielsweise Frischgas, Sauerstoff, Gasgemische, Arznei- oder Narkosemittel. Alternativ oder zusätzlich kann das Beatmungsgerät auch mehrere Ausgänge aufweisen. Atemgaseingang und/oder Atemgasausgang können einen Anschluss ausbilden oder umfassen, um jeweils einen Schlauch oder ein Schlauchsystems oder ein Patienteninterface auszuschließen. Das Gehäuse kann mehrteilig ausgebildet sein, um eine Montage zu ermöglichen. Das Gehäuse kann ein Unterteil und ein Oberteil umfassen. Das Gehäuse kann in manchen Ausführungsformen auch ein Mittelteil umfassen, das zwischen dem Unterteil und dem Oberteil angeordnet sein kann. Das Unterteil kann ein Boden sein oder einen Boden umfassen. Das Gehäuse kann ein oder mehrere Seitenwände umfassen. In manchen Ausführungen ist es auch möglich, dass Ober- und Unterteil jeweils als zwei Hälften ausgebildet sind, die zu dem einem Gehäuse zusammengesetzt werden können. Sodann kann das Ober- und/oder das Unterteil zumindest teilweise die Seitenwände umfassen.

Im durch das Gehäuse begrenzten Innenraum des Beatmungsgerätes kann - zwischen dem Atemgaseingang und dem Atemgasausgang - ein Atemgasweg des Beatmungsgerätes ausgebildet sein. Das Atemgas kann entlang des Atemgasweges geführt bzw. gefördert werden. Der Atemgasweg kann beispielsweise durch Öffnungen, Kanäle, Gänge, Kammern und dergleichen gebildet werden, aber auch durch funktionale Komponenten des Beatmungsgerätes begrenzt und/oder hindurchgeführt sein, wie beispielsweise durch den Atemgasantrieb des Beatmungsgerätes.

Das Beatmungsgerät weist die mindestens eine Kammer im Innenraum auf, die derart eingerichtet ist, dass das Gebläse bei Betrieb des Beatmungsgerätes das Atemgas aus dieser beziehen kann. Die Kammer ist somit als separate Ansaugkammer innerhalb des Beatmungsgerätes konzipiert.

Unter "Kammer" kann ein luftführender Hohlraum verstanden werden. Die Kammer weist in der Regel zwei Öffnungen auf, durch die Atemgas in die Kammer ein- oder austreten kann. Die Kammer ist ausgebildet, um Atemgas aufzunehmen und/oder abzugeben und/oder zu leiten. Hohlraum und Lumen werden hierin als Synonyme verwendet und bezeichnen das Innere von Körpern, in das Atemgas aufgenommen werden kann. Die Kammer weist eine ein Lumen der Kammer umschließende Kammerwand auf. Die Kammer ist - mit Ausnahme Ihrer Öffnungen - atemgasdicht vom restlichen Innenraum des Beatmungsgerätes abgedichtet. Die Kammer steht über ihre Öffnungen atemgasleitend mit den anderen Komponenten des Beatmungsgerätes in Verbindung. Gemäß bevorzugten Ausführungsformen sind in der Kammer keine anderen Komponenten des Beatmungsgerätes (vollständig) untergebracht. Insbesondere das Gebläse kann sich im Innenraum befinden aber nicht innerhalb der Kammer, aus der es Atemgas beziehen kann.

Die Kammer bietet den Vorteil, dass die Atemgasströmung zumindest teilweise nicht direkt auf das Gehäuse des Beatmungsgerätes trifft, so dass die Schallwellen vom Gehäuse abgehalten werden können. Zudem erfährt der Atemgasweg durch die Kammer im Inneren des Beatmungsgerätes (zusätzliche) Querschnittsänderungen und/oder Formänderungen. Die geometrische Form des Atemgasweges kann im Inneren des Beatmungsgerätes mehrfach geändert sein, so dass sich auch der Querschnitt des Atemgasweges mehrfach verkleinert oder vergrößert. Dabei ist es bevorzugt, wenn sich der Querschnitt des Atemgaswegs an einem Übergang zwischen zwei Abschnitten des Atemgasweges abrupt oder sprunghaft verkleinert oder vergrößert. In einer bevorzugten Ausführungsform ist der Übergang zwischen einzelnen Abschnitten des Atemgasweges abrupt, so dass eine deutliche geometrische Diskontinuität auftritt, was den Schall dämpft. Der Querschnitt des Atemgasweges kann im Atemgaseingang und/oder im Innenraum und/oder in der Kammer und/oder im Gebläse und/oder im Atemgasausganges abrupte Änderungen aufweisen.

Unter "Schalldämpfung" soll im Sinne der Erfindung allgemein verstanden werden, dass Schall reduziert wird. Die Reduktion des Schalls kann durch Dämpfung und/oder Dämmung und/oder akustischen Impedanzen und/oder Reflexionen und/oder Schallwellen-Überlagerung (Interferenz) und/oder Absorption erfolgen. Der Begriff "Schalldämpfung" wird hierin als Überbegriff verwendet für alle physikalischen Effekte, die den Schall reduzieren können.

Am Atemgaseingang und/oder am Atemgasausgang kann ein Flies und/oder ein Filter, beispielsweise ein Faltenfilter oder ein Filtersystem aus mehreren hintereinander angeordneten Filtern angeordnet sein. Das Flies und/oder der Filter kann eingerichtet sein, um das in das Beatmungsgerät eintretende bzw. das aus dem Beatmungsgerät austretende Atemgas zu filtern. Das Flies und/oder der Filter kann eingerichtet sein, um mögliche Feststoff-Partikel aus dem Atemgas herauszufiltern. Das Flies und/oder der Filter kann austauschbar und/oder reinigbar ausgebildet sein.

Das Beatmungsgerät umfasst das mindestens eine Gebläse. Gemäß einer Ausführungsform können die Kammer und das Gebläse separate Bauteile sein, die innerhalb des Beatmungsgerätes derart angeordnet sind, dass sie sich keine Wand mit dem Gehäuse des Beatmungsgerätes teilen.

Das Gebläse kann als Atemgasantrieb verstanden werden. Das Beatmungsgerät kann alternativ oder zusätzlich auch einen Kolbenmotor, einen Faltenbalg oder eine Ventilanordnung zum Atemgasantrieb aufweisen. Der Atemgasantrieb bzw. das Gebläse ist ausgebildet, um das Atemgas in mindestens einer Richtung anzutreiben. Der Atemgasweg kann durch das Gebläse hindurchgeführt sein. Das Gebläse ist ausgebildet, um das Atemgas vom Atemgaseingang zum Atemgasausgang zu fördern.

Das Gebläse weist in der Regel ein Gebläsegehäuse mit einer Öffnung zur Atemgasaufnahme und einer Öffnung zur Atemgasabgabe auf. Die Öffnungen können als einfache Öffnungen im Gebläsegehäuse ausgebildet sein oder als Stutzen (Saugstutzen und/oder Druckstutzen). In dem Gebläsegehäuse ist üblicherweise ein drehbar gelagertes Laufrad angeordnet sein. Dieses ist ausgebildet und angeordnet, um das Atemgas durch Rotation in das Gehäuse zu fördern sowie anschließend wieder auszustoßen und aus dem Gehäuse zu fördern. Die Rotation des Laufrads erfolgt in der Regel motorisch, vorzugsweise mittels eines Elektromotors. Eine manuell angetriebene Rotation des Laufrads ist jedoch ebenfalls denkbar. Das Gebläse wirkt so, dass das Atemgas mit einem erhöhten, vorherbestimmten Druck wieder aus dem Gehäuse ausströmbar ist. Für eine Beatmung kann das Gebläse Drehzahlen von bis zu 70.000 U/min oder sogar darüber hinaus leisten, so dass ein entsprechender Atemgas-Druck aufgebaut werden kann.

Durch das Gebläse kann der Atemgasweg in eine Saugseite und eine Druckseite unterteilt sein. Die Saugseite befindet sich stromaufwärts vor dem Gebläse. Das Atemgas kann über den Atemgaseingang in den Atemgasweg eingespeist werden und saugseitig zum Gebläse gefördert werden. Die Saugseite befindet sich somit zwischen dem Atemgaseingang und dem Gebläse. Die Druckseite befindet sich stromabwärts nach dem Gebläse. Über die Druckseite und den Atemgasausgang kann das Atemgas vom Gebläse zum Patienten befördert werden. Die Druckseite befindet sich somit zwischen dem Gebläse und dem Atemgasausgang.

Gemäß einer Ausführungsform kann das Gebläse einen Saugstutzen zur Atemgasaufnahme aufweisen. In machen Ausführungen kann der Saugstutzen bei Betrieb des Gebläses das Atemgas frei aus dem Innenraum ansaugen. Gemäß einer bevorzugten Ausführungsform kann der Saugstutzen das Atemgas bei Betrieb des Gebläses aus der Kammer ansaugen. Zu diesem Zwecke kann der Saugstutzen zur Verbindung mit der zweiten Öffnung (der Kammer) ausgebildet sein. Die Verbindung kann bevorzugt atemgasdicht ausgebildet sein.

Bei Betrieb des Gebläses kann das Atemgas von der Kammer über den Saugstutzen in das Innere des Gebläses eingeleitet werden. Der Saugstutzen kann ausgebildet sein, um das Atemgas mit einer gleichgerichteten Strömung und/oder ablösefreien Zuströmung in das Innere des Gebläses zu leiten. Der Saugstutzen kann ausgebildet sein, um eine möglichst gerade Zuströmung in das Innere des Gebläses zu gewährleisten.

Der Saugstutzen kann als einfache Öffnung im Gebläsegehäuse ausgebildet sein. Bevorzugt ist der Saugstutzen als Stutzen ausgebildet, der aus dem Gebläsegehäuse herausragend und/oder hineinragend ausgebildet ist. Sodann kann der Saugstutzen ein Volumen mit einer Länge bzw. Längsachse aufweisen, entlang derer das Atemgas geleitet werden kann. Je länger der Saugstutzen ausgebildet ist, desto besser - in Bezug auf Leistung und/oder Schall - können die Strömungseigenschaften sein. Der Saugstutzen kann eine Länge von 0 bis 30 mm aufweisen, bevorzugt von 0 bis 20 mm besonders bevorzugt von 0 bis 10mm. Der Saugstutzen kann vibrationsdämpfend mit der Kammer verbunden sein. Der Saugstutzen kann ausgebildet sein, um an ein Rohr zur Zuströmung weich entkoppelt angeschlossen zu sein, damit sich kein Körperschall überträgt. Der Saugstutzen kann als biegeschlaffes Bauteil ausgebildet sein. Der Saugstutzen kann ausgebildet sein, dass keine oder zumindest nur sehr geringe Vibrations- oder Schwingungsübertragungen auf ein angrenzendes Bauteil übertragen werden können.

Gemäß einer Ausführungsform kann das Gebläse einen Druckstutzen zur Atemgasabgabe an den Atemgasausgang aufweisen. Über den Druckstutzen kann das Atemgas aus dem Inneren des Gebläses ausgeleitet werden kann. Der Druckstutzen kann ausgebildet sein, um das Atemgas mit einem erhöhten Druck aus dem Inneren des Gebläses herauszuleiten.

Der Druckstutzen kann als einfache Öffnung im Gebläsegehäuse ausgebildet sein. Bevorzugt ist der Druckstutzen als Stutzen ausgebildet, der aus dem Gebläsegehäuse herausragend und/oder hineinragend ausgebildet ist. Sodann kann der Druckstutzen ein Volumen mit einer Länge bzw. Längsachse aufweisen, entlang derer das Atemgas geleitet werden kann. Der Druckstutzen kann einen runden, ovalen oder eckigen Querschnitt aufweisen. Der Druckstutzen weist bevorzugt einen runden Querschnitt auf.

Gemäß einer Ausführungsform kann das Gebläse derart ausgebildet sein, dass die Längsachsen von Saugstutzen und Druckstutzen dieselbe Orientierung aufweisen. Unter "Längsachse" kann diejenige Achse verstanden werden, entlang derer das Atemgas den Stutzen (in seiner Hauptrichtung) durchströmt.

Der Druckstutzen kann vibrationsdämpfend mit dem Atemgasausgang verbunden sein. Der Druckstutzen kann als biegeschlaffes Bauteil ausgebildet sein. Der Druckstutzen kann ausgebildet sein, dass keine oder zumindest nur sehr geringe Vibrations- oder Schwingungsübertragungen auf ein angrenzendes Bauteil übertragen werden können. In manchen Ausführungsformen kann es auch vorgesehen sein, dass zwischen dem Druckstutzen und dem Atemgasausgang - also druckseitig - weitere Komponenten, beispielsweise weitere Kammern und Kanäle zur Schalldämpfung und/oder zur Befeuchtung und/oder zur Erwärmung des Atemgases, angeordnet sind. Der Druckstutzen sollte in bevorzugt weich entkoppelt angeschlossen sein damit sich keine Körperschwingungen übertragen.

Das Gebläse kann als Axialgebläse oder als Radialgebläse ausgebildet sein. Bei einem Axialgebläse wird das Atemgas parallel zur Rotationsachse des Gebläses (axial) geführt. Das Atemgas kann in der gleichen Richtung aus dem Gebläse austreten, mit der es auch in das Gebläse eingetreten ist. Somit können die Längsachsen von Saugstutzen und Druckstutzen dieselbe Orientierung aufweisen.

In bevorzugten Ausführungsformen kann das Gebläse als Radialgebläse, insbesondere ein Radialgebläse mit axialem Auslass, ausgebildet sein. Bei einem Radialgebläse wird das Atemgas nach Eintritt in das Gebläse in einer Richtung senkrecht zur Rotationsachse des Gebläses (radial) geführt. Das Atemgas kann in Achsrichtung in das Gebläse eintreten und radial durch das Laufrad geführt werden. Das Gebläse kann eingerichtet sein, dass das Atemgas nach Austritt aus dem Laufrad eine erneute senkrechte Umlenkung erfährt und in den Druckstutzen geführt wird. Somit können die Längsachsen von Saugstutzen und Druckstutzen dieselbe Orientierung aufweisen.

Das bevorzugte Radialgebläse mit axialem Auslass ist besonders vorteilhaft, da sehr hohe Druckbereiche erzielt werden können. Zudem ist vorteilhaft, dass sich durch die Symmetrie des Leitapparates weniger dominante Töne ausbilden im Vergleich zu einem Radialgebläse mit radialem Auslass. Außerdem bietet es bauliche Vorteile, da Saug- und Druckstutzen beide in einer Linie axial liegen und sich Aufbauten besser packen lassen.

Gemäß einer Ausführungsform kann das Beatmungsgerät einen Kanal umfassen, der zwischen dem Atemgaseingang und der Öffnung angeordnet ist und zum Atemgasaustausch zwischen dem Atemgaseingang und der Kammer ausgebildet ist. Gemäß einer Ausführungsform kann der Kanal kann eine ein Lumen des Kanals umschließende Kanalwand umfassen. Die Kanalwand kann derart beabstandet vom Gehäuse angeordnet sein, dass ein Spalt zwischen der Kanalwand und dem Gehäuse ausgebildet ist, der verhindert, dass Schwingungen von der Kanalwand (direkt) auf das Gehäuse übertragen werden. Gemäß einer Ausführungsform kann der Kanal ein Bauteil sein, das innerhalb des Beatmungsgerätes derart angeordnet ist, dass der Kanal sich keine Wand mit dem Gehäuse des Beatmungsgerätes teilt.

Unter "Kanal" kann ein luftführender Hohlraum verstanden werden. Der Kanal weist in der Regel zwei Kanal-Öffnungen auf, durch die Atemgas in den Kanal ein- oder austreten kann. Der Kanal ist ausgebildet, um Atemgas aufzunehmen und/oder abzugeben und/oder zu leiten. Der Kanal weist zu diesem Zweck eine ein Lumen des Kanals umschließende Kanalwand auf.

Eine erste Kanal-Öffnung kann direkt mit der Öffnung der Kammer verbunden sein, so dass Kammer und Kanal in einer fluidischen Verbindung stehen. Die Kanalwand kann somit in oder an der Kammerwand enden. Eine zweite Kanal-Öffnung kann in manchen Ausführungsformen direkt mit dem Atemgaseingang des Beatmungsgerätes verbunden sein. Die Kanalwand kann sodann in oder an der Innenwand des Gehäuses enden. In diesem konkreten Ausführungsbeispiel kann der Kanal eingerichtet sein, das Atemgas direkt vom Atemgaseingang in die Kammer zu leiten.

In alternativen Ausführungsformen kann der Kanal auch mindestens mit einer Öffnung in den Innenraum des Beatmungsgerätes münden. Der Kanal kann somit eingerichtet sein, dass der Kanal über mindestens eine Kanal-- Öffnung mit dem Innenraum des Beatmungsgerätes fluidisch verbunden ist. Sodann kann der Kanal eingerichtet sein, das Atemgas aus dem Innenraum des Beatmungsgerätes in die Kammer zu leiten.

Ein Vorteil des Kanals kann sein, dass durch den Kanal eine (zusätzliche) Querschnittsänderung und/oder Formänderung des Atemgasweges vorliegt. Durch die (mehrfache) Änderung des Querschnittes und/oder der Form des Atemgasweges kann der Schall gedämpft werden. In bevorzugten Ausführungsformen können insbesondere der Innenraum und/oder die Kammer und/oder der Kanal unterschiedliche geometrische Formen aufweisen. Hierbei ist es besonders vorteilhaft, wenn Innenraum und/oder Kammer und/oder Kanal unterschiedliche Querschnitte aufweisen und der Übergang zwischen den Abschnitten des Atemgasweges abrupt oder sprunghaft verkleinert oder vergrößert werden.

Zudem kann die Schallabstrahlung nach außen hin unterdrückt werden, indem eine Übertragung der Schallwellen und/oder Schwingungen auf das Gehäuse vermieden werden. Dies kann dadurch erreicht werden, dass die einzelnen Komponenten des Beatmungsgerätes eine möglichst geringe Kontaktfläche zum Gehäuse aufweisen. Insbesondere kann die Kammerwand und/oder die Kanalwand derart beabstandet vom Gehäuse angeordnet sein, dass keine direkten Schwingungen von den Wandungen auf das Gehäuse übertragen werden. Insbesondere sollte die gesamte die Kammerwand und/oder den Kanalwand beabstandet vom Gehäuse angeordnet sein. Das Beatmungsgerät ist somit ausgebildet, dass eine direkte Schwingungsübertragung von der Kammer und/oder von dem Kanal auf das Gehäuse unterbunden ist.

Gemäß einer Ausführungsform kann der Spalt zwischen dem Gehäuse und der Kammerwand und/oder der Kanalwand eine Größe von mindestens 0,5 mm, bevorzugt von mindestens 1 mm, besonders bevorzugt von mindestens 2 mm aufweisen. Die Größe des Spaltes sollte so gewählt sein, dass keine Kollision zwischen dem Gehäuse und der Kammerwand und/oder der Kanalwand auftreten kann.

Die Größe des Spaltes und somit der Abstand zwischen dem Gehäuse und der Kammerwand und/oder der Kanalwand kann bevorzugt derart gewählt werden, dass die Kammerwand oder der Kanalwand auch dann nicht an das Gehäuse anstoßen, wenn Kammerwand oder der Kanalwand in Schwingung geraten, die beispielsweise bei Betrieb des Beatmungsgerätes durch die Atemgasströmung erzeugt werden kann.

Gemäß einer Ausführungsform kann die Kammer und/oder der Kanal aus einem nicht-porösem Material ausgebildet sein, insbesondere in einen nicht porösen Kunststoff. Die Kammer und/oder der Kanal können insbesondere ohne Schäume auskommen.

Unter "nicht-porös" kann verstanden werden, dass das Material ohne (gewollte) Lufteinschlüsse, Poren oder dergleichen ausgebildet ist. Ein nicht-poröses Material weist optimalerweise ausschließlich Merkmale eines festen Aggregatzustandes auf. Das Material kann beispielsweise ein Metall und/oder ein nicht-poröses Polymermaterial (Kunststoff) sein oder umfassen. Die Kammer und/oder der Kanal können beispielsweise mittels Gießen, Spritzgießen oder dergleichen hergestellt sein.

Das Material kann mindestens eines der folgenden metallischen Werkstoffe umfassen: Eisen; Stahl, insbesondere Edelstahl; Aluminium; Kupfer oder andere geeignete metallische Werkstoffe. Das Material kann alternativ oder zusätzlich mindestens eines der folgenden polymeren Werkstoffe umfassen: Silikon, Naturkautschuk, Polyvinylchlorid (PVC), Polymethylmethacrylat (PMMA) oder andere geeignete Kunststoffe.

Gemäß einer Ausführungsform kann die Kammer und/oder der Kanal ein elastisches Material umfassen, wobei das elastische Material die Kammerwand und/oder die Kanalwand vorzugsweise derart umhüllt, dass Körperschwingungen der Kammer und/oder des Kanals gedämpft werden. Gemäß einer Ausführungsform kann die Kammer und/oder der Kanal ein Elastomer umfassen, wobei das Elastomer die Kammerwand und/oder die Kanalwand vorzugsweise derart umhüllt, dass Körperschwingungen der Kammer und/oder des Kanals gedämpft werden.

Körperschwingungen der Kammer und/oder des Kanals können bei Betrieb des Beatmungsgerätes entstehen, wenn das Atemgas auf die Kammer und/oder Kanalwand trifft. Das elastische Material / Elastomer kann ausgebildet sein, um die Schwingungen, die durch das in die Kammer oder den Kanal auftreffende Atemgas entstehen, zu reduzieren. Dadurch wird die Möglichkeit, dass die Kammer oder Kanalwand doch auf das Gehäuse trifft, reduziert.

Gemäß einer Ausführungsform kann der Innenraum ein Volumen mit einer Tiefe, einer Breite und einer Höhe aufweisen. Die Tiefe des Innenraumes kann 80 mm bis 120 mm betragen, bevorzugt 90 mm bis 110 mm, besonders bevorzugt 100 mm bis 110 mm. Die Breite des Innenraumes kann 80 mm bis 130 mm betragen, bevorzugt 90 mm bis 120 mm, besonders bevorzugt 100 bis 110 mm. Die Höhe des Innenraumes kann 40 mm bis 110 mm betragen, bevorzugt 60 mm bis 90 mm, besonders bevorzugt unter 90 mm.

Gemäß einer Ausführungsform kann die Kammer ein Volumen mit einer Tiefe, einer Breite und einer Höhe aufweisen. Die Tiefe der Kammer kann 5 mm bis 30 mm betragen, bevorzugt 10 mm bis 20 mm, besonders bevorzugt 12 bis 16 mm. Die Breite der Kammer kann 10 mm bis 120 mm betragen, bevorzugt 20 mm bis 60 mm, besonders bevorzugt 40 mm bis 50 mm. Die Höhe der Kammer kann 40 mm bis 90 mm betragen, bevorzugt 50 mm bis 90 mm, besonders bevorzugt 70 mm bis 80 mm.

Gemäß einer Ausführungsform kann der Kanal ein Volumen mit einer Tiefe, einer Breite und einer Höhe aufweisen. Die Tiefe des Kanals kann 10 mm bis 80 mm betragen, bevorzugt 15 mm bis 60 mm, besonders bevorzugt 35 bis 46 mm. Die Breite des Kanals kann 10 mm bis 120 mm betragen, bevorzugt 20 mm bis 60 mm, besonders bevorzugt 40 mm bis 50 mm. Die Höhe des Kanals kann 1 mm bis 30 mm betragen, bevorzugt 2 mm bis 20 mm, besonders bevorzugt 4 mm bis 10 mm.

Gemäß einer Ausführungsform kann das Volumen des Innenraums mindestens 2-fach so groß wie das Volumen der Kammer sein, bevorzugt mindestens 10-fach so groß, besonders bevorzugt mindestens 20-fach so groß. Gemäß einer Ausführungsform kann das Volumen der Kammer mindestens 2-fach so groß wie das Volumen des Kanals sein, bevorzugt mindestens 3 -fach so groß, besonders bevorzugt mindestens 5-fach so groß. Gemäß bevorzugten Ausführungsformen können sich die geometrischen Abmessungen von Kammer und Kanal (deutlich) voneinander unterscheiden. Somit können Kammer und Kanal unterschiedliche akustische Impedanzen aufweisen.

Gemäß einer Ausführungsform kann die Kanalwand in den Innenraum und/oder in die Kammer hineinragend ausgebildet sein. Die Kanalwand kann somit in manchen Ausführungsformen durch die Kammerwand hindurchgeführt sein. Sodann kann der Kanal zumindest teilweise in der Kammer angeordnet sein. Die erste Kanal-Öffnung kann somit innerhalb der Kammer angeordnet sein. Auf diese Weise kann ein vorteilhafter Verlauf des Atemgases erzielt und ein Strömungsrauschen vermindert werden.

Der Kanal kann in seiner Durchflussrichtung gerade oder gebogener oder abgewinkelt verlaufen. Ein gerader Verlauf bietet fertigungstechnische Vorteile. Ein gebogener oder abgewinkelter Verlauf bildet hingegen den Vorteil, dass der Kanal besonders platzsparend im Gerät untergebracht sein kann. Der Kanal kann in seiner Durchflussrichtung auch sich trichterförmig verengend oder aufweitend verlaufen. Der Querschnitt des Kanals kann eine bevorzugt eine eckige Form aufweisen. Andere Geometrische Formen des Querschnitts sind auch denkbar, wie beispielsweise eine runde, ovale, sternförmige Form und dergleichen.

Gemäß einer Ausführungsform können die Längsachsen von Saugstutzen und/oder Druckstutzen dieselbe Orientierung aufweisen wie eine Längsachse des Kanals.

Gemäß einer Ausführungsform kann zwischen dem Atemgaseingang und dem Atemgasausgang ein Atemgasweg zum Leiten des Atemgases ausgebildet sein. Der Atemgasweg kann derart ausgebildet sein, dass das Atemgas mindestens einmal, bevorzugt mindestens zweimal, besonders bevorzugt mehrfach umgelenkt wird. Der Atemgasweg kann derart ausgebildet sein, dass das Atemgas mindestens einmal U-förmig umgelenkt wird.

Gemäß einer Ausführungsform kann das Beatmungsgerät mindestens ein Befestigungssystem umfassen, das eingerichtet ist, um die Kammer und/oder den Kanal und/oder das Gebläse mit dem Gehäuse zu verbinden, wobei das Befestigungssystem derart ausgebildet ist, dass zwischen dem Gehäuse und der Kammer ein Kontakt ausgebildet ist, der möglichst gering ist. Die Kontaktfläche zwischen dem Gehäuse und der Kammer kann unter 10 % der Gesamtfläche des Gehäuses umfassen, bevorzugt unter 5 %, besonders bevorzugt unter 2 %. Gemäß einer Ausführungsform kann das Befestigungssystem eingerichtet sein, um die Kammer und/oder den Kanal punktuell fest mit dem Gehäuse zu verbinden.

Gemäß einer Ausführungsform kann das Befestigungssystem derart ausgebildet sein, dass zwischen dem Gehäuse und dem Kanal kein direkter Kontakt besteht und der Kanal über eine Verbindung mit der Kammer in dem Beatmungsgerät gelagert ist.

Gemäß einer Ausführungsform kann das Befestigungssystem derart ausgebildet sein, dass die Verbindung zwischen Kammer und/oder Kanal und dem Gehäuse reversibel ist, so dass die Kammer und/oder Kanal zu Reinigungszwecken aus dem Beatmungsgerät entnehmbar ist. Zu diesem Zweck kann das Befestigungssystem eine Schraube und/oder einen Befestigungsclip oder dergleichen umfassen, die derart ausgebildet sind, dass die Befestigung am Gehäuse reversibel ausgebildet ist. Kammer und/oder Kanal können somit derart ausgebildet sein, dass sie zur Reinigung und/oder Desinfektion aus dem Beatmungsgerät entnehmbar sind.

In manchen Ausführungsformen können Kanal und Kammer als eine Einheit ausgebildet sein. Unter einer "Einheit" kann in diesem Falle verstanden werden, dass die Kanalwand und die Kammerwand direkt ineinander übergehen können. Die Kanalwand und die Kammerwand können aus demselben Material gefertigt sein. Die Kanalwand und die Kammerwand können in einem Fertigungsschritt gefertigt werden, beispielsweise durch Gießen oder Spritzgießen.

Die Kammer und/oder der Kanal sind vorzugsweise unabhängig vom Gehäuse ausgestaltet. Die Kammer und/oder der Kanal teilen sich somit keine Wände mit dem Gehäuse oder anderen Kammern des Beatmungsgerätes. Die Kammer und/oder der Kanal bilden eine Einheit, die separat in das Beatmungsgerät eingefügt bzw. montiert werden können.

Die Kammer und der Kanal bzw. die Einheit aus Kammer und Kanal können aus mehreren Teilen gefertigt sein. So kann beispielsweise angedacht sein, dass Kammer und/oder Kanal aus (jeweils) einem Vorderteil und einem Hinterteil gefertigt sein können, die - reversibel oder irreversibel - zu einer gebrauchsfertigen Einheit zusammengefügt werden können. Dies kann fertigungstechnische Vorteile bieten.

Derartige Vorder- und Hinterteile können beispielsweise miteinander verklebt, verschweißt, verschraubt, verklemmt, verpresst oder ähnliches werden. Vorder- und Hinterteile können beispielsweise auch mit einem Hinterschnitt miteinander verbunden werden. Die Verbindung kann reversibel oder irreversibel sein. Eine reversibel zusammengefügte Einheit kann den Vorteil bieten, dass Kammer und/oder Kanal für eine Reinigung zerlegbar sind.

Gemäß einer Ausführungsform kann das Befestigungssystem als Mittelteil ausgebildet sein und zumindest teilweise derart zwischen einem Unterteil des Gehäuses und einem Oberteil des Gehäuses positionierbar sein, dass das Oberteil und das Unterteil atemgasdicht miteinander verbunden sind.

Gemäß einer Ausführungsform kann das Mittelteil eine Hartkomponente und eine Weichkomponente umfassen, wobei die Hartkomponente zur Befestigung des Gebläses und/oder der Kammer und/oder des Kanals ausgebildet ist, wobei die Weichkomponente zur Abdichtung und/oder Vibrationsdämpfung ausgebildet ist.

Gemäß einer Ausführungsform kann das Mittelteil eine Durchführung umfassen, die eingerichtet ist, um eine Passage für Leitungen und/oder Kabel aus dem Gehäuseinneren heraus zu ermöglichen, wobei die Durchführung mindestens eine Durchführungsöffnung umfasst, die von der Weichkomponente umgeben ist.

Gemäß einer Ausführungsform kann in der Durchführungsöffnung mindestens ein Fixierelement angeordnet sein, das ausgebildet ist, um die Leitungen und/oder Kabel in den Durchführungsöffnung zu fixieren.

Gemäß einer Ausführungsform kann das Beatmungsgerät ein Gebläsehalterungssystem mit einer Gebläseaufnahme und einem Halteelement umfassen, wobei das Halteelement am Gebläse angeordnet ist und die Gebläseaufnahme eingerichtet ist, um das Halteelement zumindest teilweise derart aufzunehmen, so dass das Gebläse innerhalb des Gehäuses befestigt ist.

Gemäß einer Ausführungsform kann die Gebläseaufnahme am Mittelteil und/oder an der Kammerwand und/oder an der Kanalwand angeordnet sein. Gemäß einer Ausführungsform kann die Gebläseaufnahme durch das Mittelteil und/oder durch die Kammerwand und/oder durch die Kanalwand ausgebildet sein. Gemäß einer Ausführungsform kann die Gebläseaufnahme in das Mittelteil und/oder in die Kammerwand und/oder in die Kanalwand integriert sein.

Gemäß einer Ausführungsform kann das Halteelement eine Nabe umfassen, über die das Halteelement am Gebläse befestigt ist. Das Halteelement kann ferner eine Wand umfassen, die zumindest bereichsweise formkomplementär zur Gebläseaufnahme ausgebildet ist und über die das Halteelement in der Gebläseaufnahme gelagert ist.

Gemäß einer Ausführungsform kann zwischen der Nabe und der Wand kann eine Zwischenwand ausgebildet sein, die eine geringere Wandstärke aufweist als die Nabe und/oder die Wand. Gemäß einer Ausführungsform kann die Zwischenwand in seiner Erstreckungsrichtung von der Nabe zur Wand mindestens eine Biegung aufweisen, bevorzugt mindestens zwei, besonders bevorzugt mindestens drei Biegungen. Gemäß einer Ausführungsform kann die Zwischenwand in seiner Erstreckungsrichtung von der Nabe zur Wand eine gewellte Form aufweisen. Gemäß einer Ausführungsform kann die Zwischenwand partiell unterschiedliche Wandstärken aufweisen. Gemäß einer Ausführungsform kann die Zwischenwand mindestens eine Öffnung umfassen, bevorzugt mindestens zwei, besonders bevorzugt mindestens vier.

### Kurze Beschreibung der Zeichnungen

Im Folgenden werden Ausführungsformen der Erfindung mit Bezug auf die beigefügten Abbildungen beschrieben. Weder die Beschreibung noch die Abbildungen sind als Beschränkung des Umfangs der Erfindung zu verstehen.
Fig. 1 zeigt eine schematische Darstellung eines Beatmungsgerätes gemäß einer Ausführungsform der Erfindung.
Fig. 2, Fig. 3 und Fig. 4 zeigen perspektivische Teilansichten eines Beatmungsgerätes gemäß einer Ausführungsform der Erfindung.
Fig. 5 zeigt eine Teilansicht eines Beatmungsgerätes von oben gemäß einer Ausführungsform der Erfindung.
Fig. 6 zeigt eine Teilansicht eines Beatmungsgerätes gemäß einer Ausführungsform der Erfindung.
Fig. 7 zeigt eine perspektivische Ansicht einer Kammer und eines Kanals gemäß einer Ausführungsform der Erfindung.
Fig. 8 zeigt eine perspektivische Schnittansicht und Fig. 9 zeigt eine Schnittansicht einer Kammer und eines Kanals gemäß einer Ausführungsform der Erfindung.
Fig. 10 zeigt eine schematische Darstellung eines Beatmungsgerätes gemäß einer Ausführungsform der Erfindung.
Fig. 11 und 12 zeigen perspektivische Teilansichten eines Beatmungsgerätes gemäß einer weiteren Ausführungsform der Erfindung.
Fig. 13 zeigt einen Ausschnitt eines Mittelteils mit einer Durchführung gemäß einer Ausführungsform der Erfindung.
Fig. 14 und 15 zeigen verschiedene Ausführungsformen der Halteelemente in unterschiedlichen Ansichten.

Die Figuren sind rein schematisch und nicht maßstabsgetreu. Werden in verschiedenen Zeichnungen gleiche Bezugszeichen verwendet, so bezeichnen diese Bezugszeichen gleiche oder gleichwirkende Merkmale.

### Ausführungsformen der Erfindung

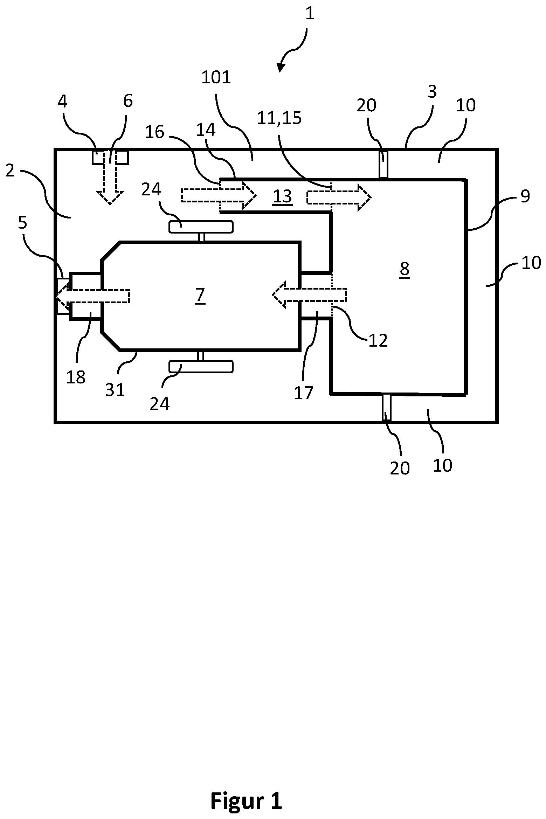

Fig. 1 zeigt eine schematische Darstellung eines Beatmungsgerätes 1 gemäß einer Ausführungsform der Erfindung. Das Beatmungsgerät 1 umfasst ein Gehäuse 3, das einen Innenraum 2 des Beatmungsgerätes 1 nach außen hin begrenzt. Das Gehäuse 3 ist eingerichtet, den Innenraum 2 im Wesentlichen atemgasdicht von der Umgebungsluft abzugrenzen.

Das Beatmungsgerät 1 umfasst jedoch zumindest einen Atemgaseingang 4, über den ein Atemgas in den Innenraum 2 des Beatmungsgerätes 1 hineingelangen kann. Der Atemgaseingang 4 kann am Gehäuse 3 angeordnet sein und/oder durch das Gehäuse 3 ausgebildet sein. Der Atemgaseingang 4 kann als einfache Öffnung im Gehäuse 3 ausgebildet sein. In manchen Ausführungsformen kann der Atemgaseingang 4 auch als Anschluss ausgebildet sein, über den ein Schlauch oder dergleichen an das Beatmungsgerät angeschlossen werden kann (nicht gezeigt).

Zudem umfasst das Beatmungsgerät 1 einen Atemgasausgang 5, über den das Atemgas aus dem Innenraum 2 des Beatmungsgerätes 1 herausgelangen kann. Der Atemgasausgang 5 kann am Gehäuse 3 angeordnet sein und/oder durch das Gehäuse 3 ausgebildet sein. Der Atemgasausgang 5 kann als einfache Öffnung in dem Gehäuse 3 ausgebildet sein. In manchen Ausführungsformen kann der Atemgasausgang 5 auch als Anschluss ausgebildet sein, über den ein Schlauch, ein Schlauchsystem, ein Befeuchtungssystem und/oder ein Patienteninterface angeschlossen werden kann (nicht gezeigt).

Am Atemgaseingang 4 und/oder am Atemgasausgang 5 kann ein hier nicht dargestellter Filter und/oder ein Flies angeordnet sein, um das in das Beatmungsgerät eintretende oder aus dem Beatmungsgerät austretende Atemgas zu filtern.

Im Innenraum 2 ist - zwischen dem Atemgaseingang 4 und dem Atemgasausgang 5 - ein Atemgasweg 6 ausgebildet. Der Atemgasweg 6 ist schematisch durch gestrichelte Blockpfeile angedeutet. Die Richtung, mit der das Atemgas in der Regel entlang des Atemgasweges 6 durch den Innenraum 2 geführt ist, ist in den Abbildungen durch die Pfeilrichtung des gestrichelten Blockpfeils dargestellt. Aus der Abbildung wird ersichtlich, dass der Atemgasweg 6 mindestens eine Umlenkung erfährt, bevorzugt zwei, besonders bevorzugt drei oder mehr. In besonders bevorzugten Ausführungsformen wird das Atemgas vielfach umgelenkt.

Das Beatmungsgerät 1 umfasst einen im Innenraum 2 angeordneten Atemgasantrieb, der als Gebläse 7 ausgebildet sein kann. Das Gebläse 7 umfasst in der Regel ein Gebläsegehäuse 31, mit dem das Gebläseinnere vom Innenraum 2 abgegrenzt ist. Der Atemgasweg ist durch das Gebläse 7 hindurch geleitet. Das Gebläse 7 ist ausgebildet, um das Atemgas in mindestens einer Richtung anzutreiben. Das Gebläse 7 ist ausgebildet, um das Atemgas vom Atemgaseingang 4 zum Atemgasausgang 5 zu fördern. Zu diesem Zweck kann das Gebläse 7 einen Saugstutzen 17 und einen Druckstutzen 18 aufweisen. Saugstutzen 17 und/oder Druckstutzen 18 können beispielsweise durch das Gebläsegehäuse 31 ausgebildet sein oder an dieses angeformt sein.

Das Atemgas kann über den Saugstutzen 17 in das Innere des Gebläses 7 eintreten und über den Druckstutzen 18 wieder aus dem Gebläse 7 austreten. Die Längsachsen sind durch die Durchflussrichtung des Atemgases definiert (und entsprechen in der Abbildung den Blockpfeilen über Saugstutzen 17 bzw. Druckstutzen 18). Die Längsachsen von Saugstutzen 17 und Druckstutzen 18 können - wie hier dargestellt - dieselbe Orientierung aufweisen. Sodann kann die Durchflussrichtung des Atemgases durch Saugstutzen und Druckstutzen dieselbe sein.

Im Inneren des Gebläses 7 kann ein (hier nicht dargestelltes) drehbar gelagertes Laufrad angeordnet sein, das von einem (ebenfalls nicht dargestellten) Motor angetrieben werden kann. Die Rotationsbewegung des Laufrades erzeugt eine Atemgas-Strömung. Dadurch kann Atemgas durch den Saugstutzen 17 in das Innere des Gebläses 7 eingesaugt werden und beschleunigt und/oder komprimiert durch den Druckstutzen 18 wieder abgegeben werden. Somit kann der Atemgasweg in eine Saugseite und eine Druckseite unterteilt sein.

Die Saugseite befindet sich stromaufwärts vor dem Saugstutzen 17 des Gebläses 7. Das Atemgas kann über den Atemgaseingang 4 in den Atemgasweg eingespeist werden und saugseitig zum Gebläse 7 gefördert werden. Die Saugseite befindet sich somit zwischen dem Atemgaseingang 4 und dem Saugstutzen 17 des Gebläses 7.

Die Druckseite befindet sich stromabwärts nach dem Druckstutzen 18 des Gebläses 7. Über die Druckseite und den Atemgasausgang 5 kann das Atemgas vom Gebläse 7 zum Patienten befördert werden. Die Druckseite befindet sich somit zwischen dem Druckstutzen 18 des Gebläses 7 und dem Atemgasausgang 5. Der Druckstutzen 18 kann, wie in Fig. 1 skizziert, direkt mit dem Atemgasausgang 5 verbunden sein. Zwischen dem Druckstutzen 18 und dem Atemgasausgang 5 können jedoch auch ein oder mehrere weitere Komponenten angeordnet sein, wie beispielsweise weitere Kammern und Kanäle zur Schalldämpfung und/oder zur Befeuchtung und/oder zur Erwärmung des Atemgases (nicht gezeigt).

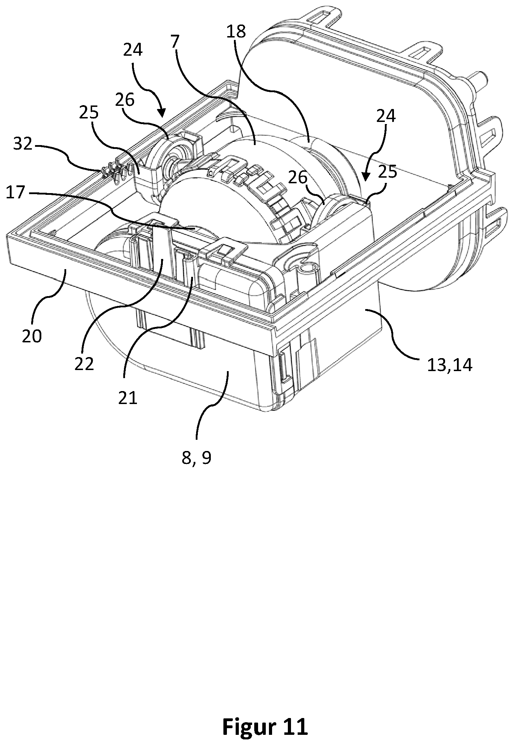

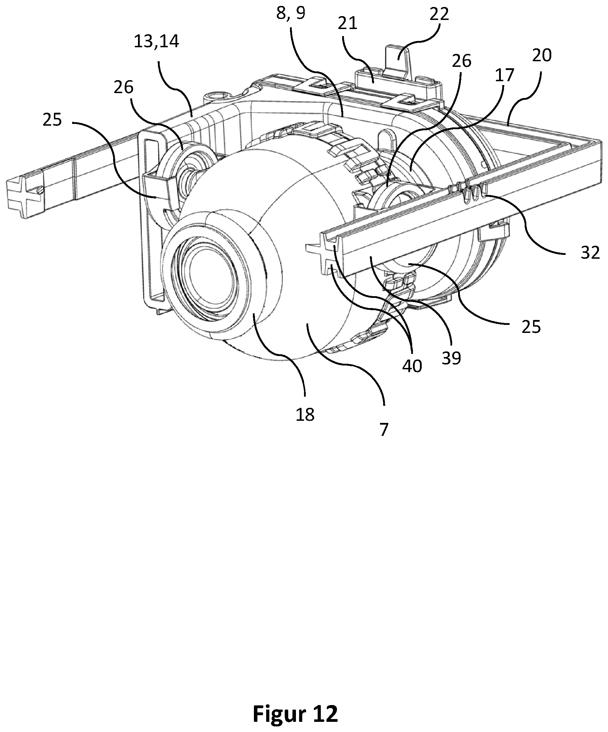

Das Gebläse 7 kann über mindestens ein Gebläsehalterungssystem 24 an dem Gehäuse 3 befestigt sein. Das Gebläse 7 kann über das Gebläsehalterungssystem 24 direkt an dem Gehäuse befestigt sein. In alternativen Ausführungsformen kann das Gebläse auch indirekt an dem Gehäuse befestigt sein, beispielsweise durch Zwischenschaltung mindestens eines weiteren Bauteils 20 (siehe Fig. 11 und 12). In Fig. 1 sind zwei Gebläsehalterungssysteme 24 skizziert, die eingerichtet sind, um das Gebläse 7 punktuell fest mit dem Gehäuse 3 zu verbinden. Mehrere Gebläsehalterungssysteme 24 sind auch denkbar. Hierbei ist jedoch darauf zu achten, dass die Kontaktfläche zwischen Gebläse 7 und Gehäuse 3 möglichst gering ist, so dass die Schwingungsübertragung vom Gebläse 7 auf das Gehäuse 3 minimiert ist. Saugstutzen 17 und/oder Druckstutzen 18 können ebenfalls dazu ausgebildet sein, dass Gebläse 7 innerhalb des Gehäuses 3 zu lagern bzw. in Position zu halten.

Im Innenraum 2 ist eine Kammer 8 angeordnet. Die Kammer 8 umfasst eine ein Lumen der Kammer begrenzende Kammerwand 9. Die Kammer 8 umfasst eine Öffnung 11 zum Atemgasaustausch mit dem Atemgaseingang 4. Die Kammer 8 ist somit atemgasleitend mit dem Atemgaseingang 4 verbunden. Die Kammer 8 umfasst eine zweite Öffnung 12 zum Atemgasaustausch mit dem Gebläse 7. Das Gebläse 7 ist eingerichtet, um Atemgas über die Öffnung 12 aus der Kammer 8 anzusaugen und zum Atemgasausgang 5 zu fördern. Dazu kann der Saugstutzen 17 zur atemgasdichten Verbindung mit der zweiten Öffnung 12 ausgebildet sein.

Die Kammerwand 9 ist derart beabstandet vom Gehäuse 3 angeordnet, dass keine direkten Schwingungen von der Kammerwand 9 auf das Gehäuse 3 übertragen werden. Zwischen Kammerwand 9 und Gehäuse 3 ist ein Spalt 10 ausgebildet, der atemgasführend ist. Der Spalt 10 ist im Wesentlichen leer, und insbesondere nicht mit einem (porösen) Material ausgefüllt. Der Innenraum 3 bzw. der Spalt 10 ist vorzugsweise schaumfrei ausgebildet.

Aus Fig. 1 ist ersichtlich, dass das Beatmungsgerät zusätzlich einen Kanal 13 mit einer ein Lumen des Kanals begrenzenden Kanalwand 14 umfassen kann. Die Kanalwand 14 kann ebenso wie die Kammerwand 9 derart beabstandet vom Gehäuse 3 angeordnet sein, dass keine direkten Schwingungen von der Kanalwand 14 auf das Gehäuse 3 übertragen werden. Zwischen Kanalwand 14 und Gehäuse 3 ist vorzugsweise ein Spalt 101 ausgebildet, der atemgasführend ist. Der Spalt 101 ist im Wesentlichen leer, und insbesondere nicht mit einem (porösen) Material ausgefüllt. Sowohl Innenraum 3 als auch die Spalte 10, 101 sind vorzugsweise schaumfrei ausgebildet. Der Spalt 10 bzw. 101 umgibt die Kammer 9 und/oder den Kanal 13 vorzugsweise vollständig.

Der Kanal 13 kann eine erste Kanal-Öffnung 15 aufweisen, die wie gezeigt direkt mit der Öffnung 11 (der Kammer) verbunden sein kann. Kanalwand 14 und Kammerwand 9 können somit direkt ineinander übergehen. Kammer 8 und Kanal 13 stehen somit in einer fluidischen Verbindung. Der Kanal 13 kann eine zweite Kanal-Öffnung 16 aufweisen, die in fluidischer Verbindung mit dem Atemgaseingang 4 steht. Im Ausführungsbeispiel gemäß Fig. 1 mündet der Kanal 13 mit seiner zweiten Kanal-- Öffnung 16 in den Innenraum 2. Der Kanal 13 ist somit eingerichtet, das Atemgas aus dem Innenraum 2 in die Kammer 8 zu leiten. Die Kammer 8 und der Kanal 13 können aus einem nichtporösen Material ausgebildet sein. Vorzugsweise ist der gesamte Atemgasweg nicht in Kontakt mit einem porösen Material, also schaumfrei ausgebildet. Das Atemgas kann durch den Kanal 13 in die Kammer 8 und von der Kammer 8 in das Gebläse 7 geführt sein. Dabei kann der Atemgasweg 6 derart ausgebildet sein, dass das Atemgas mehrfach umgelenkt wird. Das Atemgas kann beispielsweise mindestens einmal U-förmig umgelenkt werden.

Das Beatmungsgerät 1 kann mindestens ein Befestigungssystem 20 zur Befestigung der Kammer 8 und/oder des Kanals 13 im Beatmungsgerät 1 umfassen. Das Befestigungssystem 20 kann auch ausgebildet sein, um andere Komponenten des Beatmungsgerätes 1 zu befestigen, beispielsweise das Gebläse 7. In Fig. 1 sind zwei Befestigungssysteme 20 skizziert, die eingerichtet sind, um die Kammer 8 und/oder den Kanal 13 punktuell fest mit dem Gehäuse 3 zu verbinden. Mehrere Befestigungssysteme 20 sind auch denkbar. Hierbei ist jedoch darauf zu achten, dass ein Kontakt zwischen Kammer 8 und/oder Kanal 13 und Gehäuse 3 möglichst gering ist, so dass die Schwingungsübertragung auf das Gehäuse 3 minimiert ist. Zusätzlich oder alternativ kann vorgesehen sein, dass das Befestigungssystem 20 oder eine Kontaktfläche zwischen Gehäuse und Befestigungssystem 20 ein Schwingungsdämpfendes Material umfassen oder aus einem solchen gefertigt sind. Die Befestigungssysteme 20 können an der Kammer 8 und/oder an dem Kanal 13 ansetzen. In Fig. 1 ist exemplarisch gezeigt, dass zwei Befestigungssysteme 20 an der Kammer 8 ansetzen und die Kammer 8 und den mit der Kammer 8 verbundenen Kanal 13 am Gehäuse 3 befestigen. Das Befestigungssystem 20 kann in manchen Ausführungsformen auch als ein Bauteil ausgebildet sein, das zwischen einem Unterteil und einem Oberteil des Gehäuses 3 angeordnet sein kann. Das Befestigungssystem 20 kann sodann als Mittelteil 20 zwischen Unterteil und Oberteil ausgebildet sein (siehe Fig. 11 und 12). Das Mittelteil 20 kann zumindest teilweise ein Teil des Gehäuses darstellen.

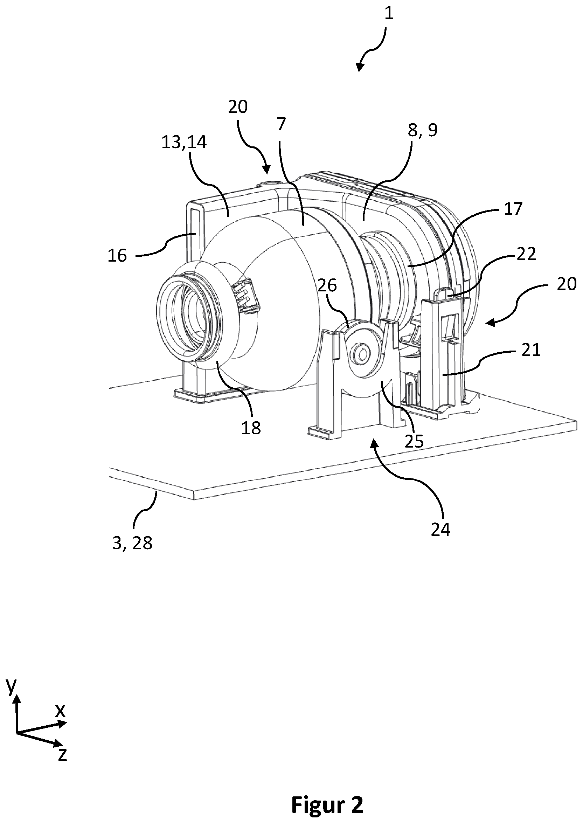

**Fig. 2****,** **Fig. 3** **und** **Fig. 4** zeigen unterschiedliche perspektivische Teilansichten eines Beatmungsgerätes 1 gemäß einer Ausführungsform der Erfindung. Zur besseren Darstellbarkeit ist das Gehäuse 3 bis auf einen Boden 28 nicht dargestellt.

Aus Fig. 2 wird ersichtlich, dass das Gebläse 7 über das Gebläsehalterungssystem 24 an dem Gehäuse 3 befestigt sein kann, in diesem Falle an einem Boden 28 des Gehäuses 3. Das Gebläse 7 kann in alternativen Ausführungsformen ebenso an einer oder mehreren Seitenwänden 29 des Gehäuses und/oder am Oberteil 30 des Gehäuses befestigt sein. Das Gebläse 7 kann in alternativen Ausführungsformen ebenso, zumindest teilweise, am Mittelteil 20 befestigt sein (siehe Fig. 11 und 12). In dem hier dargestellten konkreten Ausführungsbeispiel ist das Gebläsehalterungssystem 24 nicht an derjenigen Gehäusewand befestigt, an der der (hier nicht darstellbare) Atemgaseingang 4 und/oder Atemgasausgang 5 angeordnet ist.

Das Gebläse 7 kann über mindestens ein, vorzugsweise zwei Gebläsehalterungssysteme 24 gehaltert sein. Mehrere Gebläsehalterungssysteme 24 sind auch denkbar. Alternativ oder zusätzlich kann das Gebläse 7 auch über den Saugstutzen 17 und/oder den Druckstutzen 18 gehaltert oder zumindest in Position gehalten werden. In einer konkreten Ausführungsbeispiel kann das Gebläse 7 über zwei Gebläsehalterungssysteme 24 am Gehäuse 3 befestigt sein und zusätzlich über den Saugstutzen 17 und den Druckstutzen 18 in Position gehalten werden.

Das Gebläsehalterungssystem 24 kann ausgebildet sein, um das Gebläse 7 beabstandet vom Gehäuse 3 im Innenraum 2 des Beatmungsgerätes 1 zu haltern. Das Gebläse 7 kann somit frei im Innenraum 2 gelagert sein. Das Gebläse 7 kann derart im Innenraum 2 gelagert sein, dass es bei Betrieb des Beatmungsgerätes von (saugseitigem) Atemgas umströmt werden kann. Das Gebläsehalterungssystem 24 ist vorzugsweise derart ausgebildet, dass das Gebläse 7 zur Reinigung und/oder Desinfizierung aus dem Beatmungsgerät 1 entnehmbar ist.

Das Gebläsehalterungssystem 24 kann eine Gebläseaufnahme 25 umfassen. Die Gebläseaufnahme 25 kann direkt oder indirekt am Gehäuse 3 befestigt sein. In dem dargestellten Ausführungsbeispiel ist die Gebläseaufnahme 25 an dem Boden 28 des Gehäuses 3 befestigt. In alternativen Ausführungsformen kann die Befestigung auch an den Seitenwänden 29 des Gehäuses und/oder am Oberteil 30 des Gehäuses 3 ansetzen (nicht gezeigt). In alternativen Ausführungsformen kann die Gebläseaufnahme 25 auch, zumindest teilweise, durch das Mittelteil 20 ausgebildet bzw. in dieses integriert sein oder an diesem angeordnet sein. Zudem kann das Gebläsehalterungssystem 24 ein Halteelement 26 umfassen. Das Halteelement 26 kann direkt oder indirekt am Gebläse 7 befestigt sein. Gebläseaufnahme 25 und Halteelement 26 können miteinander verbindbar ausgebildet sein. Zu diesem Zweck können Gebläseaufnahme 25 und Halteelement 26 zumindest bereichsweise formkomplementär ausgebildet sein. In dem hier dargestellten Ausführungsbeispiel kann die Gebläseaufnahme 25 einen Aufnahmeraum ausbilden, in den das Halteelement 26 aufgenommen werden kann. Hier kann das Halteelement 26 in manchen Ausführungsformen fixiert werden, um einen sicheren Halt des Gebläses 3 zu gewährleisten. Es ist jedoch auch denkbar, dass das Gebläse 3 über das Halteelement 26 einfach in die Gebläseaufnahme 25 eingehängt wird.

Gebläseaufnahme 25 und/oder Halteelement 26 können aus einem vibrationsdämpfenden Material gefertigt sein oder ein solches umfassen. Beispielsweise können Gebläseaufnahme 25 und/oder Halteelement 26 zumindest an ihren Kontaktflächen ein vibrationsdämpfendes Material, beispielsweise Silikon, umfassen.

Aus Fig. 2 wird ferner ersichtlich, dass die Kammer 8 und/oder der Kanal 14 über das Befestigungssystem 20 an dem Gehäuse 3 befestigt sein kann, in diesem Falle am Boden 28 des Gehäuses 3. Kammer 8 und/oder Kanal 14 können in alternativen Ausführungsformen ebenso an einer oder mehreren Seitenwänden 29 des Gehäuses und/oder am Oberteil 30 des Gehäuses 3 befestigt sein. In dem hier dargestellten konkreten Ausführungsbeispiel ist das Befestigungssystem 20 nicht an derjenigen Gehäusewand befestigt, an der der (hier nicht darstellbare) Atemgaseingang 4 und/oder der Atemgasausgang 5 angeordnet ist. Kammer 8 und/oder Kanal 14 können über mindestens ein, vorzugsweise zwei Befestigungssysteme 20 gehaltert sein. Mehrere Befestigungssysteme 20 sind auch denkbar.

Das Befestigungssystem 20 kann ausgebildet sein, um die Kammer 8 und/oder den Kanal 14 beabstandet vom Gehäuse 3 im Innenraum 2 des Beatmungsgerätes 1 zu haltern. Kammer 8 und/oder Kanal 14 können somit frei im Innenraum 2 gelagert sein. Kammer 8 und/oder Kanal 14 können derart im Innenraum 2 gelagert sein, dass sie bei Betrieb des Beatmungsgerätes von (saugseitigem) Atemgas umströmt werden können.

Die Kammer 8 ist derart im Innenraum 2 gelagert, dass die Kammerwand 9 beabstandet vom Gehäuse 3 angeordnet ist. Zwischen der Kammerwand 9 und dem Gehäuse 3 ist ein Spalt 10 ausgebildet, der verhindert, dass Schwingungen von der Kammerwand 9 (direkt) auf das Gehäuse 3 übertragen werden. In diesem konkreten Ausführungsbeispiel umfasst das Beatmungsgerät 1 neben der Kammer 8 auch einen Kanal 13, der an die Kammer 8 angeformt ist. Kammerwand 9 und Kanalwand 14 gehen in diesem Falle direkt ineinander über. Somit bilden Kammer 8 und Kanal 13 eine Einheit, die in das Innere des Beatmungsgerätes 1 einsetzbar ist.

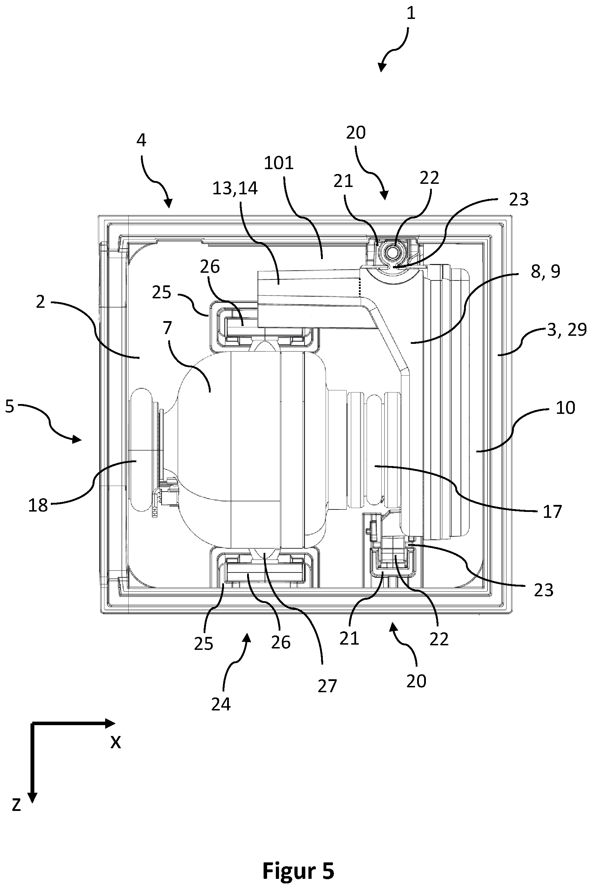

Auch der Kanal 14 kann vorzugsweise derart im Innenraum 2 gelagert sein, dass die Kanalwand 14 beabstandet vom Gehäuse 3 angeordnet ist. Zwischen der Kanalwand 14 und dem Gehäuse 3 kann ebenfalls ein Spalt 101 ausgebildet sein, der verhindert, dass Schwingungen von der Kanalwand 14 (direkt) auf das Gehäuse 3 übertragen werden (siehe Fig. 5). Kammer 8 und/oder dem Kanal 14 können aus einem vibrationsdämpfenden Material gefertigt sein oder ein solches umfassen. Beispielsweise können Kammer 8 und/oder Kanal 14 zumindest von einem vibrationsdämpfenden Material, beispielsweise Silikon, ummantelt sein. Dies kann die Körperschwingungen unterbinden oder zumindest mindern.

Das Befestigungssystem 20 ist vorzugsweise derart ausgebildet, dass Kammer 8 und/oder Kanal 14 zur Reinigung und/oder Desinfizierung aus dem Beatmungsgerät 1 entnehmbar (und wieder einsetzbar) sind. Das Befestigungssystem 20 ist demnach derart ausgebildet, die Kammer 8 und/oder den Kanal 13 reversibel an dem Beatmungsgerät 1 zu befestigen.

Das Befestigungssystem 20 kann eine Befestigungsaufnahme 21 umfassen. Die Befestigungsaufnahme 21 kann direkt oder indirekt am Gehäuse 3 befestigt sein. In dem dargestellten Ausführungsbeispiel ist die Befestigungsaufnahme 21 am Boden 28 des Gehäuses 3 befestigt. Eine Anordnung der Befestigungsaufnahme 20 an einer oder mehreren Seitenwänden 29 oder am Oberteil 30 oder an anderen Bauteilen ist in alternativen Ausführungsformen ebenfalls möglich. Zudem kann das Befestigungssystem 20 ein Befestigungselement 22 umfassen. Das Befestigungselement 22 kann direkt oder indirekt am der Kammer 8 und/oder dem Kanal 14 befestigt sein. Befestigungsaufnahme 21 und Befestigungselement 22 können miteinander verbindbar ausgebildet sein. Zu diesem Zweck können Befestigungsaufnahme 21 und Befestigungselement 22 zumindest bereichsweise formkomplementär ausgebildet sein. In dem hier dargestellten Ausführungsbeispiel kann die Befestigungsaufnahme 21 einen Aufnahmeraum ausbilden, in den das Befestigungselement 22aufgenommen werden kann.

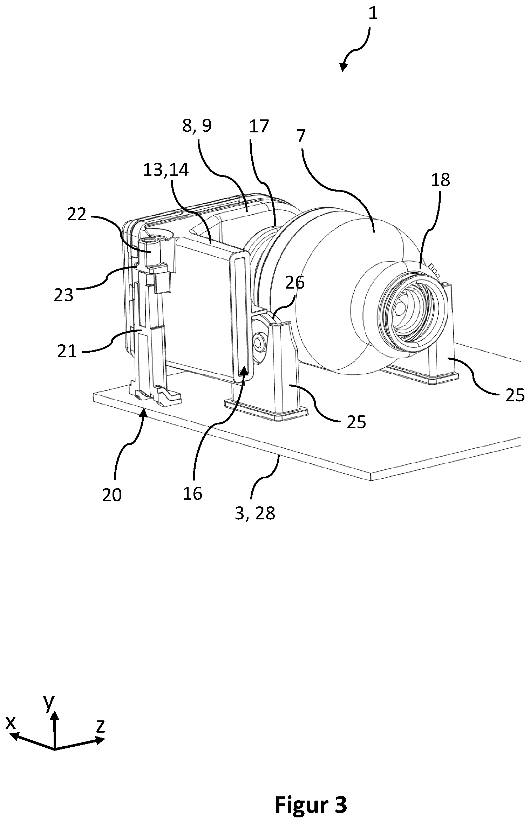

Befestigungsaufnahme 21 und Befestigungselement 22 können zu einer Steckverbindung verbindbar ausgebildet sein. Hierbei ist es von Vorteil, wenn die Verbindung drehfest ausgebildet ist. Sodann kann die Kammer 8 und/oder der Kanal 13 punktuell fest an dem Gehäuse 3 gelagert sein und keine (Dreh)Beweglichkeit aufweisen. Die mehreren Befestigungssysteme 20 können identisch ausgebildet sein. In manchen Ausführungsformen ist es denkbar, dass die Befestigungssysteme 20 unterschiedlich ausgebildet sein können (vergleiche Fig. 2 und Fig. 3).

In Fig. 2 ist ein Befestigungssystem 20 dargestellt, bei dem das Befestigungselement 22 als Clip ausgebildet ist, der in die Befestigungsaufnahme 21 reversibel eingesteckt werden kann. Das als Clip ausgebildete Befestigungselement 22 hat eine Außenkontur, die zumindest bereichsweise in eine formkomplementäre Innenkontur der Befestigungsaufnahme 21 gesteckt werden kann. Das als Clip ausgebildete Befestigungselement 22 kann ein Bedienelement umfassen, mit dessen Betätigung es wieder aus der Befestigungsaufnahme 22 gelöst werden kann. Dies bietet ein besonders einfaches und sicheres Befestigungssystem.

In Fig. 3 ist ein Befestigungssystem 20 dargestellt, bei dem das Befestigungselement 22 auf die Befestigungsaufnahme 21 gesteckt werden kann und über eine Schraube, einen Haltestift oder dergleichen fixiert werden kann (nicht gezeigt). Dies bietet eine besonders sichere Befestigung.

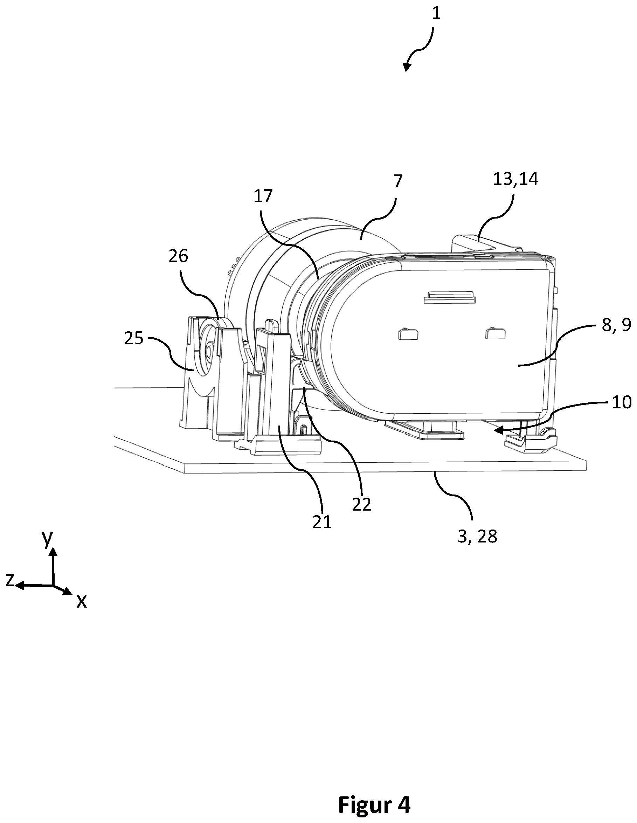

Das Befestigungssystem 20 kann bevorzugt derart ausgebildet ist, dass zwischen dem Gehäuse 3 und der Kammer 8 ein Kontakt 23 ausgebildet ist, der möglichst gering ist. Die Kammer 8 ist - bis auf den Kontakt 23 ringsherum beabstandet zum Gehäuse 3 angeordnet. Zwischen der Kammerwand 9 und dem Gehäuse 3 ist ein Spalt 10 ausgebildet, der verhindert, dass Schwingungen von der Kammerwand 9 direkt auf das Gehäuse 3 übertragen werden. Aus Fig 4 geht hervor, dass dieser Spalt 10 somit auch zwischen der Kammerwand 9 und dem Boden 28 des Gehäuses 3 ausgebildet ist.

Fig. 5 zeigt eine Teilansicht eines Beatmungsgerätes von oben gemäß einer Ausführungsform der Erfindung. Aus Fig. 5 wird ersichtlich, dass die Kammer 8 auch lateral beabstandet zum Gehäuse 3 angeordnet ist und ein Spalt 10 zwischen der Kammerwand 9 und dem Gehäuse 3 ausgebildet ist. Der Spalt 10 ist somit auch zwischen der Kammerwand 9 und den Seitenwänden 29 des Gehäuses 3 ausgebildet.

Ähnlich kann es sich in Bezug auf einen potenziellen Kanal 13 verhalten. Der Kanal 13 kann lateral beabstandet zum Gehäuse 3 angeordnet sein. Zwischen der Kanalwand 14 und dem Gehäuse 3 kann ein Spalt 101 ausgebildet sein. Der Spalt 101 ist somit auch zwischen der Kammerwand 9 und den Seitenwänden 29 des Gehäuses 3 ausgebildet.

In den Ausführungsformen gemäß den Figuren weist der Kanal 13 keinen (direkten) Kontakt zum Gehäuse 3 auf. Der Kanal 13 kann, wie gezeigt, mit der Kammer 8 verbunden sein und über diese indirekt an dem Gehäuse 3 befestigt sein. In der gezeigten Ausführungsform weist das Beatmungsgerät 1 somit ein Befestigungssystem 20 auf, über das die Kammer 8 direkt an dem Gehäuse 3 befestigt sein kann. In alternativen Ausführungsformen ist es auch denkbar, dass - alternativ oder zusätzlich - das Befestigungssystem 20 eingerichtet ist, den Kanal 13 an dem Gehäuse 3 zu befestigen (nicht gezeigt)

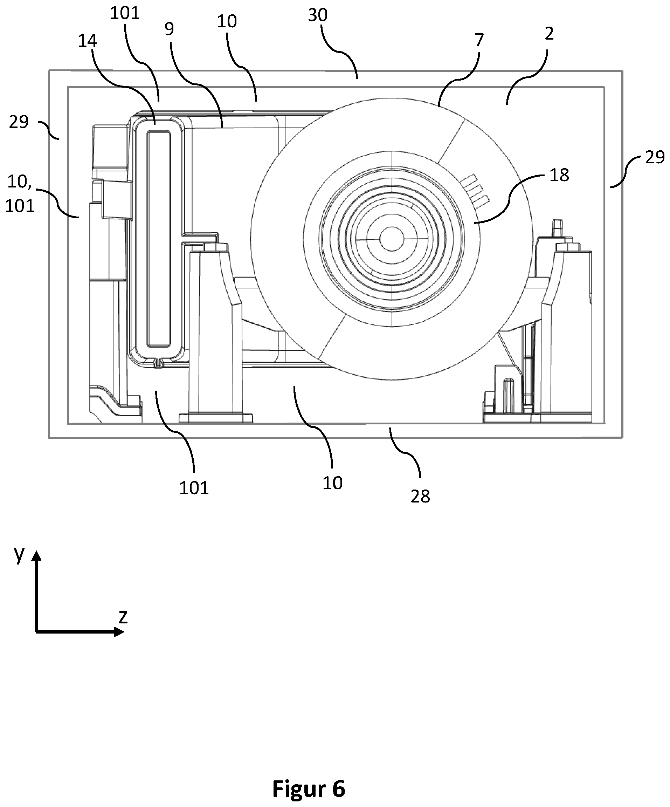

Fig. 6 zeigt eine Teilansicht eines Beatmungsgerätes gemäß einer Ausführungsform der Erfindung. Diese Ansicht verdeutlicht, dass der Spalt 10 zwischen der Kammerwand 9 und dem Gehäuse 3 auch zum hier gezeigten Boden 28, zum Oberteil 30 und zu den Seitenwänden 29 des Gehäuses 3 hin ausgebildet ist. Ebenso wird ersichtlich, dass zwischen der Kanalwand 14 und der Seitenwand 29, dem Boden 28 und dem Oberteil 30 ebenfalls ein Spalt 101 ausgebildet ist. Die Kanalwand 14 steht in diesem Ausführungsbeispiel nicht in einem direkten Kontakt zum Gehäuse 3.

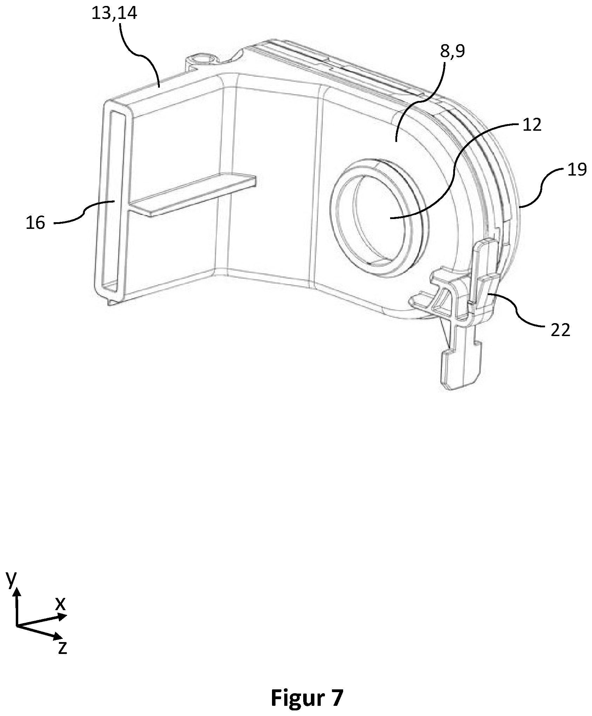

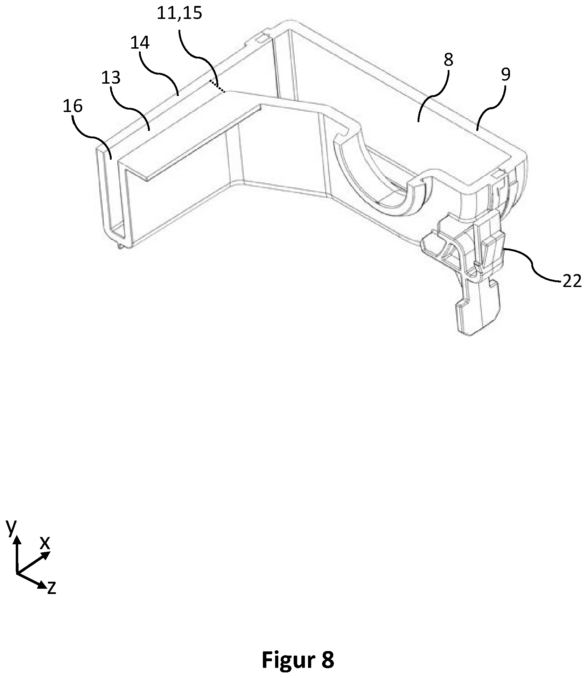

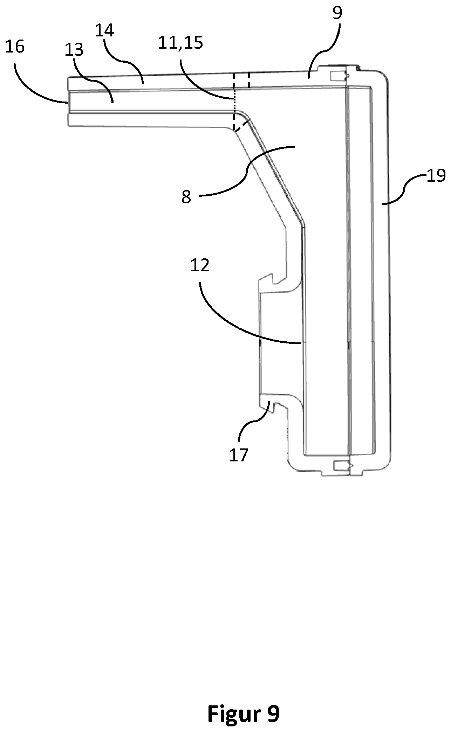

**Fig.** 7 zeigt eine perspektivische Ansicht einer Kammer 8 und eines Kanals 13 gemäß einer Ausführungsform der Erfindung und **Fig.** 8 zeigt eine perspektivische Schnittansicht einer Kammer 8 und eines Kanals 13 gemäß einer Ausführungsform der Erfindung und **Fig.** 9 zeigt eine Schnittansicht einer Kammer und eines Kanals gemäß einer Ausführungsform der Erfindung.

Aus den Fig. 7, 8 und 9 wird deutlich, dass Kammer 8 und Kanal 13 als Einheit ausgebildet sein können. Die Kammerwand 9 und die Kanalwand 14 können somit direkt ineinander übergehen. Dadurch kann eine Öffnung 11 der Kammer direkt benachbart zur ersten Kanal-Öffnung 15 angeordnet sein. Kammer 8 und Kanal 13 stehen über die Öffnungen 11,15 in einer direkten fluidischen Verbindung.

Die Einheit aus Kammer 8 und Kanal 13 kann ausgebildet sein, in das Beatmungsgerät 1 einsetzbar und wieder entnehmbar zu sein. Um eine bessere Reinigung zu gewähren, kann diese Einheit aus mindestens zwei Einzelteilen zu bestehen, so dass die Kammer 8 und/oder der Kanal 13 zu Reinigungszwecken geöffnet werden können. Hier dargestellt ist exemplarisch, dass eine Rückwand 19 der Kammer von dem Rest der Einheit lösbar ausgestaltet sein kann, um die Kammer 8 zu Reinigungszwecken zu öffnen. In alternativen Ausführungsformen ist auch denkbar, dass die Einheit ohne einen Kanal auskommt und die Öffnung 11 der Kammer einen direkten Übergang vom Innenraum 2 zum Lumen der Kammer 8 darstellt (nicht gezeigt).

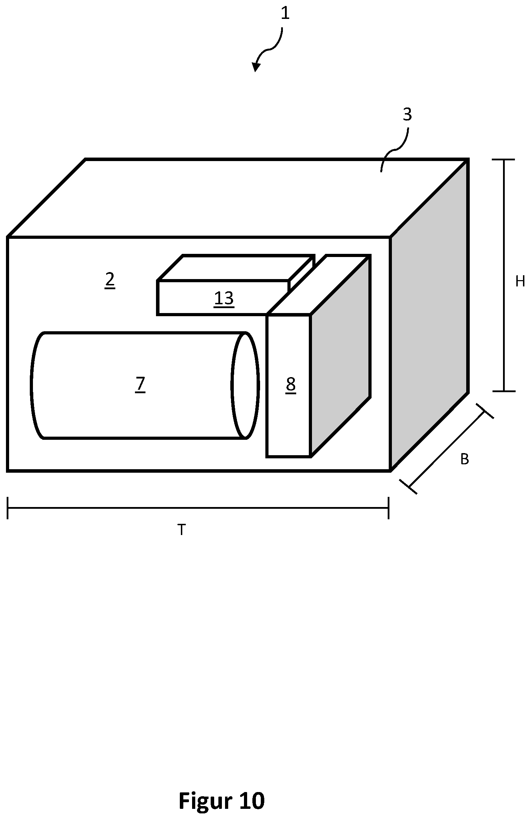

**Fig. 10** zeigt eine schematische Darstellung eines Beatmungsgerätes gemäß einer Ausführungsform der Erfindung. Aus Fig. 10 wird ersichtlich, dass das Beatmungsgerät 1 bzw. der Innenraum 2, die Kammer 8 und der Kanal 13 Volumen mit einer Tiefe T, einer Breite B und einer Höhe H aufweisen. Das Volumen des Innenraums 2 kann mindestens 2-fach so groß wie das Volumen der Kammer 8 sein, bevorzugt mindestens 20-fach so groß. Das Volumen der Kammer 8 kann mindestens 2-fach so groß wie das Volumen des Kanals 13 sein, bevorzugt mindestens 5-fach so groß. Die geometrischen Abmessungen von Kammer 8 und Kanal 13 können sich deutlich voneinander unterscheiden. Somit können Kammer 8 und Kanal 13 unterschiedliche akustische Impedanzen aufweisen.

**Fig. 11** **und** **12** zeigen unterschiedliche perspektivische Teilansichten eines Beatmungsgerätes 1 gemäß einer weiteren Ausführungsform der Erfindung. Zur besseren Darstellbarkeit sind weite Teile des Gehäuses nicht dargestellt. In der Ausführungsform gemäß Fig. 11 und 12 umfasst das Beatmungsgerät 1 ein Befestigungssystem 20, das als Mittelteil 20 ausgebildet ist. Das Mittelteil 20 kann zumindest teilweise ein Teil des Gehäuses ausbilden bzw. sein.

Aus Fig. 11 und 12 wird ersichtlich, wie das Gebläse 7, die Kammer 8 und der Kanal 13 in Bezug zum Mittelteil 20 angeordnet sein können. Das Mittelteil 20 kann als tragendes und/oder abdichtendes Bauteil ausgebildet sein. Das Mittelteil 20 kann im Wesentlichen als Rahmen ausgebildet sein, der zwischen einem hier nicht dargestellten Oberteil und einem Unterteil des Gehäuses angeordnet sein kann. Das Mittelteil 20 kann sodann als ein Teil des Gehäuses angesehen werden. Das Mittelteil 20 kann sodann den Vorteil bieten, dass das Oberteil und das Unterteil atemgasdicht miteinander verbindbar sind. Hierbei kann der Rahmen des Mittelteils 20 eine abdichtende Funktion übernehmen.

Ferner kann das Mittelteil 20 ein oder mehrere Bauteile des Beatmungsgerätes 1 aufnehmen. Das Mittelteil 20 kann als Befestigungssystem 20 ausgebildet sein oder dieses umfassen. Am Mittelteil 20 können sodann die Kammer 8 und/oder der Kanal 13 und/oder das Gebläse 7 befestigt sein. Die Befestigung ist bevorzugt vibrationsdämpfend ausgebildet.

Das Mittelteil 20 kann ausgebildet sein, um zumindest das Gebläse 7 und/oder den Kanal 13 und/oder die Kammer 8 im Beatmungsgerät 1 zu haltern. Das Mittelteil 20 kann ausgebildet sein, um die Kammer 8 und/oder den Kanal 13 und/oder das Gebläse 7 beabstandet vom Gehäuse 3 im Innenraum 2 des Beatmungsgerätes 1 zu haltern. Das Mittelteil 20 kann das Befestigungssystem 20 und/oder das Gebläsehalterungssystem 24 oder Teile dessen umfassen.

Aus dem Ausführungsbeispiel gemäß der Fig. 11 und 12 wird ersichtlich, dass das Gebläsehalterungssystem 24 in das Mittelteil 20 und/oder in die Kanalwand 14 integriert oder an dem Kanal 13 angeordnet sein kann. In anderen Ausführungen wäre auch denkbar, dass das Gebläsehalterungssystem 24 in die Kammerwand 9 integriert ist oder an der Kammer 8 angeordnet sein kann.

Hier gezeigt ist ein konkretes Ausführungsbeispiel, bei dem zumindest eine der Gebläseaufnahmen 25 in das Mittelteil 20 integriert bzw. durch dieses ausgebildet ist. Eine weitere Gebläseaufnahme 25 ist in die Kanalwand 14 integriert bzw. durch diese ausgebildet. Somit kann das Gebläse 7 über die Halteelemente 26 in die Gebläseaufnahmen 25 des Mittelteils 20 und der Kanalwand 14 eingehängt werden.

Der Vorteil besteht darin, dass das Gebläse 7 bzw. die Gebläseaufnahmen 25 keinen direkten Kontakt zum (restlichen) Gehäuse 3 aufweisen. Die Gebläseaufnahmen 25 haben weder einen direkten Kontakt zum Boden 28 noch zur Seitenwand 29 oder zum Oberteil 30 oder zum Unterteil. Dies kann einen besonderen effektiven Schutz vor einer Vibrations- und somit Schallübertragung auf das Gehäuse 3 bieten.

Aus dem Ausführungsbeispiel gemäß der Fig. 11 und 12 wird ferner ersichtlich, dass die Kammer 8 und/oder der Kanal 13 am Mittelteil 20 befestigt sein kann. Das Mittelteil 20 kann wie gezeigt die Befestigungsaufnahme 21 umfassen oder ausbilden. An der Kammerwand 9 und/oder der Kanalwand 14 kann ein entsprechendes Befestigungselement 22 ausgebildet sein, die in die Befestigungsaufnahme 21 eingreifen können. Somit können die Kammer 8 und/oder der Kanal 13 am Mittelteil 20 befestigt werden. Die Befestigung erfolgt reversibel oder irreversibel. Eine reversible Befestigung bietet den Vorteil, dass die Komponenten des Beatmungsgerätes zu Zwecken der Reinigung oder Reparatur auseinandernehmbar ausgebildet sein können.

Das Mittelteil 20 kann vorteilhaft für eine Montage des Beatmungsgerätes 1 sein. Das Mittelteil 20 kann eine Hartkomponente 39 und/oder eine Weichkomponente 40 umfassen, bevorzugt eine Hartkomponente 39 und eine Weichkomponente 40 (siehe Fig. 12). Bevorzugt wird das Mittelteil 20 im 2-Komponenten-Spritzguss gefertigt, dies erleichtert die Herstellung. Die Hartkomponente 39 kann beispielsweise Kunststoff sein oder umfassen. Die Weichkomponente 40 kann beispielsweise ein thermoplastisches Elastomer (TPE) oder Silikon sein oder umfassen. Das Mittelteil 20 umfasst somit eine angespritzte Dichtung 40, was die Herstellung und/oder Montage des Beatmungsgerätes wesentlich vereinfacht. Die Hartkomponente 39 kann das Mittelteil 20 in Form halten. Die Hartkomponente 39 kann aus dem gleichen Material wie das Gehäuse gefertigt sein. Die Hartkomponente 39 kann sodann als Teil des Gehäuses angesehen werden. Die Weichkomponente 40 kann eine abdichtende Funktion und/oder eine vibrationsdämpfende Funktion aufweisen. Somit sind keine zusätzlichen Dichtungselemente, insbesondere keine biegeschlaffen Dichtungen nötig. Dies ermöglicht eine schlanke Montage.

Das Beatmungsgerät 1 kann Module aufweisen, die vormontierbar sein können. Das Mittelteil 20 kann ein erstes Modul sein. Die Kammer 8 oder die Kammer 8 und der Kanal 13 können ein zweites Modul sein. Das Gebläse 7, vorzugweise mit dem Saugstutzen 17 und dem Druckstutzen und den Halteelementen 26, kann ein drittes Modul sein. Diese Module können miteinander zu einem Gesamtmodul verbindbar ausgebildet sein. Das Mittelteil 20 bietet den Vorteil, dass eine Vormontage des Gesamtmoduls erfolgen kann. Ein solches Gesamtmodul kann sodann in das Gehäuse 3, beispielsweise in ein Unterteil des Gehäuses, eingesetzt werden. Das Gesamtmodul kann neben dem Mittelteil 20 zumindest das Gebläse 7 und die Kammer 8 umfassen. Ferner kann das Modul den Kanal 13 umfassen. Ein solches Gesamtmodul ist beispielsweise in Fig. 11 gezeigt.

Das Gebläse 7 kann dabei bevorzugt bereits den Saugstutzen 17 und/oder den Druckstutzen 18 aufweisen. Der Saugstutzen 17 kann in der Vormontage mit der Kammer 8 verbunden werden. Der Druckstutzen 18 kann in der Vormontage mit dem Mittelteil 20 verbunden werden. Am Gebläse 7 kann auch bereits das mindestens eine Halteelement26, bevorzugt zwei Halteelemente 26 vormontiert sein, die in die Halteaufnahmen 25 eingreifen, um das Gebläse sicher zu befestigen. Die Halteaufnahmen 25 können wie hier dargestellt am Mittelteil 20 und an dem Kanal 13 angeordnet sein. In alternativen Ausführungen ist eine Anordnung an der Kammer auch denkbar. Das Gesamtmodul kann sodann schwingungsfrei mit dem hier nicht dargestellten Gehäuse verbunden werden.

Das Mittelteil 20 kann ferner mindestens eine Durchführung 32 umfassen. Die Durchführung 32 kann ausgebildet sein, um eine Passage für Leitungen und/oder Kabel zu ermöglichen. Dadurch können Leitungen und/oder Kabel aus dem Inneren des Gehäuses nach außerhalb geführt werden, wobei die Leitungen/Kabel vor Beschädigung geschützt sind.

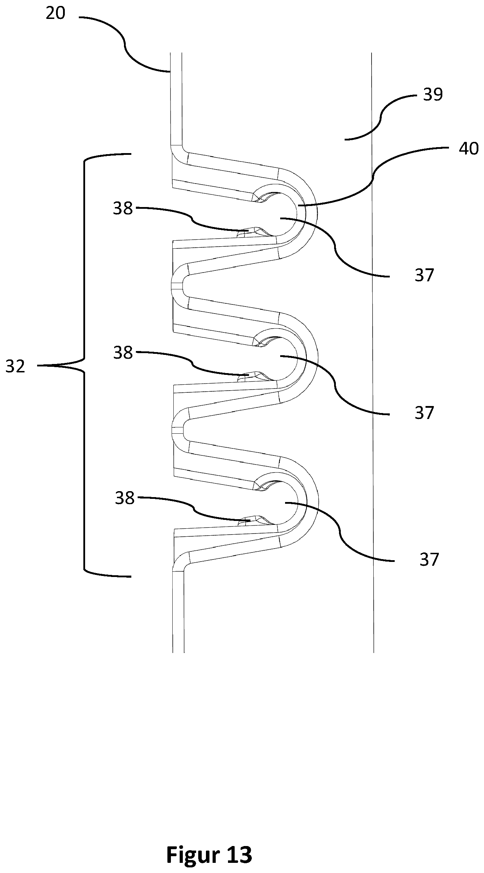

**Fig.** 13 zeigt einen Ausschnitt des Mittelteils 20, der eine solche Durchführung 32 aufweist. Die Durchführung 32 kann mindestens eine Durchführungsöffnungen 37 aufweisen. Die Durchführung 32 kann in manchen Ausführungen auch mindestens zwei, bevorzugt drei oder mehr Durchführungsöffnungen 37 aufweisen. In dem konkreten Ausführungsbeispiel gemäß Fig. 13 weist die Durchführung 32 drei Durchführungsöffnungen 37 auf. Die Durchführungsöffnungen 37 können ausgebildet sein, um eine Passage für Leitungen und/oder Kabel zu ermöglichen.

Beispielsweise kann die Durchführung 32 ausgebildet sein, um eine hier nicht gezeigte elektrische Leitung des Gebläses 7 zumindest teilweise aufzunehmen. Die Leitung des Gebläses kann mindestens ein elektrisches Kabel, Draht oder Litzen umfassen. Das Mittelteil 20 stellt hierbei die Dichtung für die Gebläseleitung im Bereich der Durchführung 32 dar. Dadurch können die Leitungen oder Kabel im Material des Mittelteils 20 eingebettet sein.

Die Leitung des Gebläses kann in manchen Ausführungen auch als Flachbandleitung ausgebildet sein, beispielsweise als FFC-Leitung. Sodann können die Leitungen einfach zwischen dem Oberteil und dem Unterteil oder zwischen dem Mittelteil und dem Unterteil oder zwischen dem Mittelteil und dem Oberteil eingeklemmt sein, insbesondere bei Verwendung von FFC-Leitungen (nicht gezeigt).

Die Durchführungsöffnungen 37 können von der Weichkomponente 40 begrenzt sein. Die Durchführungsöffnungen 37 können somit von einem Dichtmaterial, beispielsweise TPE oder Silikon, begrenzt und in diese eingebettet sein. In oder an den Durchführungsöffnungen 37 können Fixierelemente 38 angeordnet sein. Die Fixierelemente 38 können ausgebildet sein, um die Leitungen oder Kabel in den Durchführungsöffnung 37 zu fixieren. Somit können die Leitungen oder Kabel auch dann in der Durchführungsöffnung 37 verbleiben, wenn das Gehäuse noch nicht zusammenmontiert ist. Die Fixierelemente 38 können aus demselben Material ausgebildet sein, wie die Weichkomponente 40 bzw. durch diese geformt sein.

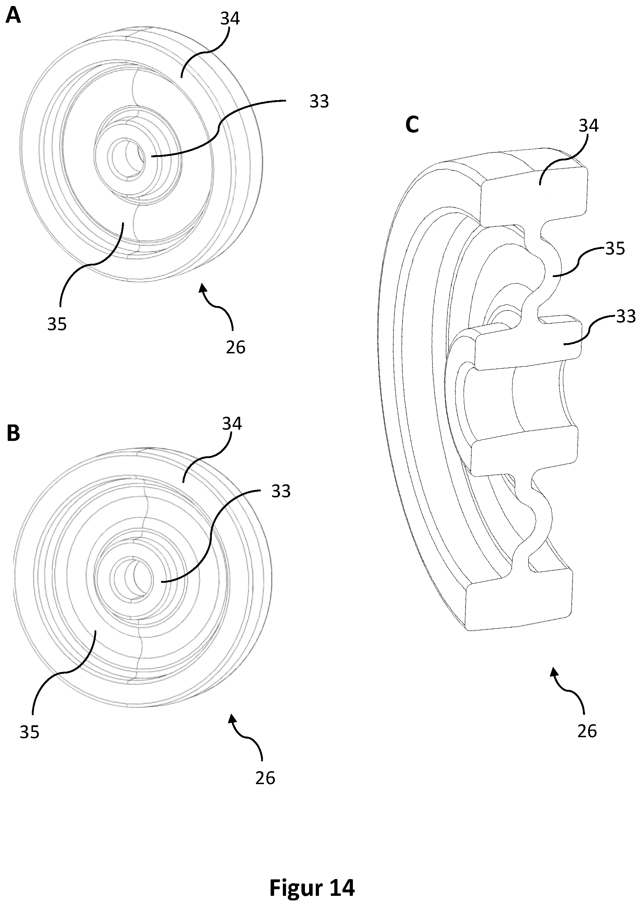

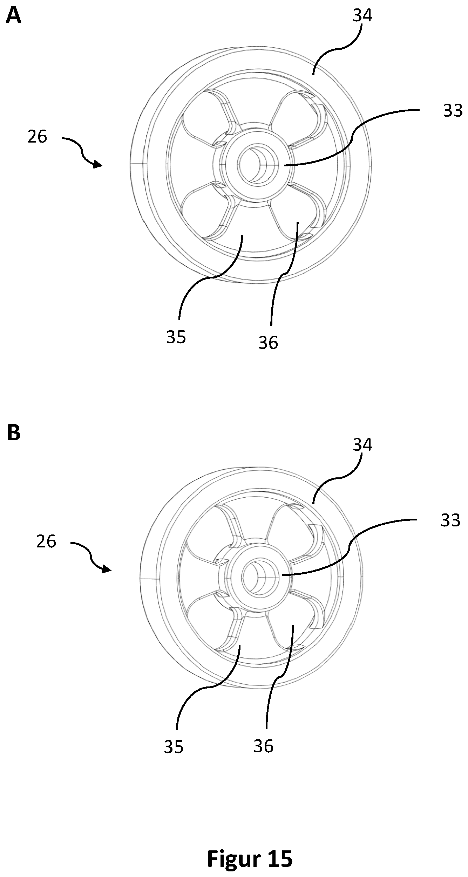

**Fig. 14** **und** **15** zeigen verschiedene Ausführungsformen der Halteelemente 26. Fig. 14A zeigt eine perspektivische Ansicht von vorne, Fig. 14B eine perspektivische Ansicht von hinten und Fig. 14C eine Schnittansicht einer ersten Ausführungsform. Fig. 15A zeigt eine weiteren Ausführungsform in einer perspektivischen Ansicht von vorne. Fig. 15B zeigt eine alternative Ausführungsform in einer perspektivischen Ansicht von vorne.

Die Halteelemente 26 können zumindest bereichsweise formkomplementär zur Gebläseaufnahme 25 ausgebildet sein. Die Halteelemente 26 können wie gezeigt eine runde Außenkontur aufweisen. Entsprechend formkomplementär können die Gebläseaufnahmen 25 ausgebildet sein, um die Halteelemente 26 aufzunehmen. Die Halteelemente 26 können aus einem vibrationsdämpfenden Material gefertigt sein oder ein solches umfassen. Die Halteelemente 26 können beispielsweise aus Silikon gefertigt sein. Es ist auch denkbar, dass die Halteelemente 26 nur an ihren Kontaktflächen, also an ihrer Außenkontur, Silikon umfassen. Das vibrationsdämpfende Material bietet den Vorteil, dass Vibrationen des Gebläses 7 sich nicht oder nur stark vermindert auf die Gebläseaufnahmen 25 übertragen können. Das Gebläse 7 kann somit abgefedert befestigt sein.

Die Halteelemente 26 können beispielsweise scheibenförmig oder rädchenförmig ausgebildet sein. Die Halteelemente 26 können eine Nabe 33 umfassen. Die Nabe 33 kann ausgebildet sein, um mit dem Gebläse 7 verbindbar zu sein. Die Verbindung zwischen Gebläse 7 und Halteelement 25 kann über nicht gezeigte Elemente am Gebläse 7 realisiert sein, die in die Nabe 33 einführbar ausgebildet sein können.

Die Halteelemente 26 können eine Wand 34 umfassen. Die Wand 34 kann die Außenkontur der Halteelemente 26 bestimmen. Die Wand 34 kann aus Silikon oder einem anderen vibrationsdämpfenden Material gefertigt sein. Die Wand 34 kann wie gezeigt eine runde, insbesondere eine kreisrunde Außenkontur aufweisen, die formkomplementär zur Gebläseaufnahme 25 ist. Dies bietet den Vorteil einer besonders einfachen Montage. Somit muss keine Ausrichtung stattfinden. In anderen Ausführungen ist auch eine ovale oder eine eckige Außenkontur möglich (nicht gezeigt). Nabe 33 und Wand 34 können, wie aus Fig. 14C ersichtlich wird, eine gleiche oder ähnliche Wandstärke aufweisen. In anderen Ausführungen ist es auch denkbar, dass die Nabe 33 dicker oder dünner ausgebildet ist als die Wand 34.

Die Halteelemente 26 können eine Zwischenwand 35 umfassen. Die Zwischenwand 35 kann zwischen der Wand 34 und der Nabe 33 ausgebildet sein. Die Zwischenwand 35 kann eine gleiche Wandstärke aufweisen wie die Wand 34 und/oder die Nabe 33 (nicht gezeigt). Die Zwischenwand 35 kann auch eine dickere Wandstärke aufweisen als die Wand 34 (nicht gezeigt) oder eine dünnere Wandstärke (siehe Fig. 14C). Die Zwischenwand 35 kann zwischen der Wand 34 und der Nabe 33 gerade ausgebildet sein (siehe Fig. 14). Die Zwischenwand 35 kann zwischen der Wand 34 und der Nabe 33 auch mindestens eine Biegung aufweisen, bevorzugt mindestens zwei, besonders bevorzugt mindestens drei (siehe Fig. 14). Die Zwischenwand 35 kann, wie aus Fig. 14C deutlich wird, eine gewellte Form aufweisen. Die Zwischenwand kann mindestens einmal, bevorzugt mindestens zweimal, besonders bevorzugt mindestens dreimal konvex bzw. konkav gebogen ausgebildet sein. Im Vergleich zu einer geraden Zwischenwand 35 bietet eine gewellte Form einen besonders guten Schwingungsausgleich, sowohl bei radialer als auch axialer Bewegung.

Die Zwischenwand 35 kann, wie in Fig. 14C gezeigt, eine konstante Wandstärke aufweisen. Eine gewellte Form der Zwischenwand 35 mit konstanter Wandstärke bietet den Vorteil, dass die Halteelement 26 eine hohe Lebensdauer und Stabilität aufweisen. Die Halteelemente 26 haben vorteilhafter eine drehsymmetrische Form. Dadurch können die Halteelemente 26 ein gleichmäßiges Schwindungsverhalten im Herstellungsprozess aufweisen.

Die Zwischenwand 35 kann in alternativen Ausführungen auch partiell unterschiedliche Wandstärken aufweisen (nicht gezeigt). Die Zwischenwand 35 kann wie in Fig. 15 gezeigt mindestens eine Öffnung 36 umfassen, bevorzugt mindestens 2, besonders bevorzugt mindestens 4. Die Öffnungen 36 können ungleichmäßig, oder bevorzugt gleichmäßig, verteilt in oder an der Zwischenwand 35 vorliegen.

Die Größe der Öffnungen 36 kann unterschiedlich ausgebildet sein. Fig. 15A zeigt eine Ausführung mit kleineren Öffnungen 36, Fig. 15B zeigt eine Ausführungsform mit größeren Öffnungen 36. Die Öffnungen 36 können mindestens 5 % der Gesamtfläche der Zwischenwand 35 ausmachen, bevorzugt mindestens 15 %, besonders bevorzugt mindestens 30 % oder mehr. Die Öffnungen 36 können jegliche Form aufweisen, von eckig über oval bis rund oder wie gezeigt tropfenförmig. Die Öffnungen 36 können als (vollständiger) Durchbruch in der Zwischenwand 35 ausgebildet sein. Die Öffnungen 36 können auch als verdünnte oder verdickte Bereiche in der Zwischenwand 35 ausgebildet sein.

Die Halteelemente 26 können am Gebläse 7 vormontiert werden, so dass das Gebläse 7 mit den Halteelementen 26 in die Gebläseaufnahme 25 eingesetzt werden kann. Die runde Außenkontur der Halteelemente 26 erleichtert die Ausrichtung des Gebläses 7 in der Gebläseaufnahme 25.

Durch die Öffnungen 36 kann eine Materialersparnis und somit auch eine Kostenersparnis erreicht werden. Durch die Öffnungen 36 können insbesondere auch Drehbewegungen um die Nabe 33 besser abgefedert werden, die durch Vibrationen oder andere Bewegungen des Gebläses entstehen können. Dabei kann sich das Material der Wand 34 und/oder der Zwischenwand 35 in die Öffnungen 36 verformen. Dies begünstigt die vibrationsdämpfende Befestigung des Gebläses 7 innerhalb des Beatmungsgerätes 1.

Abschließend wird darauf hingewiesen, dass Begriffe wie "aufweisen", "umfassen", "einschließen", "mit" usw. keine anderen Elemente oder Schritte ausschließen und unbestimmte Artikel wie "ein" oder "eine" keine Vielzahl ausschließen.

Ferner wird darauf hingewiesen, dass Merkmale oder Schritte, die mit Verweis auf eine der vorstehenden Ausführungsformen beschrieben sind, auch in Kombination mit Merkmalen oder Schritten, die mit Verweis auf andere der vorstehenden Ausführungsformen beschrieben sind, verwendet werden können.

Bezugszeichen in den Ansprüchen sind nicht als Beschränkung des Umfangs des durch die Ansprüche definierten Gegenstands zu verstehen.

### Bezugszeichenliste

- 1: Beatmungsgerät
- 2: Innenraum
- 3: Gehäuse
- 4: Atemgaseingang
- 5: Atemgasausgang
- 6: Atemgasweg
- 7: Gebläse (Atemgasantrieb)
- 8: Kammer
- 9: Kammerwand
- 10: Spalt
- 11: Öffnung
- 12: Zweite Öffnung
- 13: Kanal
- 14: Kanalwand
- 15: Erste Kanal-Öffnung
- 16: Zweite Kanal-Öffnung
- 17: Saugstutzen
- 18: Druckstutzen
- 19: Rückwand
- 20: Befestigungssystem / Mittelteil
- 21: Befestigungsaufnahme
- 22: Befestigungselement
- 23: Kontakt
- 24: Gebläsehalterungssystem
- 25: Gebläseaufnahme
- 26: Halteelement
- 27: Kontakt
- 28: Boden
- 29: Seitenwand
- 30: Oberteil
- 31: Gebläsegehäuse
- 32: Durchführung
- 33: Nabe
- 34: Wand
- 35: Zwischenwand
- 36: Öffnung
- 37: Durchführungsöffnung
- 38: Fixierelement
- 39: Hartkomponente
- 40: Weichkomponente
- 101: Spalt
- B: Breite
- H: Höhe
- T: Tiefe

## Patentansprüche

1. Beatmungsgerät (1) umfassend
- ein einen Innenraum (2) des Beatmungsgerätes (1) begrenzendes Gehäuse (3) mit einem Atemgaseingang (4) und einem Atemgasausgang (5),
- ein im Innenraum (2) angeordnetes Gebläse (7), das ausgebildet ist, um Atemgas vom Atemgaseingang (4) zum Atemgasausgang (5) zu fördern,
- eine im Innenraum (2) angeordnete Kammer (8), die eine Öffnung (11) zum Atemgasaustausch mit dem Atemgaseingang (4) und eine zweite Öffnung (12) zum Atemgasaustausch mit dem Gebläse (7) umfasst,
wobei das Gebläse (7) eingerichtet ist, um Atemgas über die zweite Öffnung (12) aus der Kammer (8) anzusaugen und zum Atemgasausgang (5) zu fördern,
wobei die Kammer (8) eine ein Lumen der Kammer umschließende Kammerwand (9) umfasst, die derart beabstandet vom Gehäuse (3) angeordnet ist, dass ein Spalt (10) zwischen der Kammerwand (9) und dem Gehäuse (3) ausgebildet ist, der verhindert, dass Schwingungen von der Kammerwand (9) auf das Gehäuse (3) übertragen werden.

2. Beatmungsgerät (1) nach Anspruch 1, wobei die Kammer (8) und das Gebläse (7) separate Bauteile sind, die innerhalb des Beatmungsgerätes (1) derart angeordnet sind, dass sie sich keine Wand mit dem Gehäuse (3) des Beatmungsgerätes (1) teilen.

3. Beatmungsgerät (1) nach einem der vorhergehenden Ansprüche, wobei das Gebläse (7) einen Saugstutzen (17) zur Atemgasaufnahme aufweist, wobei der Saugstutzen (17) zur atemgasdichten Verbindung mit der zweiten Öffnung (12) ausgebildet ist und/oder wobei das Gebläse (7) einen Druckstutzen (18) zur Atemgasabgabe an den Atemgasausgang (5) aufweist, wobei das Gebläse (7) derart ausgebildet ist, dass die Längsachsen von Saugstutzen (17) und Druckstutzen (18) dieselbe Orientierung aufweisen.

4. Beatmungsgerät (1) nach einem der vorhergehenden Ansprüche, wobei das Beatmungsgerät (1) einen Kanal (13) umfasst, der zwischen dem Atemgaseingang (3) und der Öffnung (11) angeordnet ist und zum Atemgasaustausch zwischen dem Atemgaseingang (3) und der Kammer (8) ausgebildet ist.

5. Beatmungsgerät (1) nach Anspruch 4, wobei der Kanal (13) eine ein Lumen des Kanals umschließende Kanalwand (14) umfasst, wobei die Kanalwand (14) derart beabstandet vom Gehäuse (3) angeordnet ist, dass ein Spalt (101) zwischen der Kanalwand (14) und dem Gehäuse (3) ausgebildet ist, der verhindert, dass Schwingungen von der Kanalwand (14) auf das Gehäuse (3) übertragen werden und/oder wobei der Kanal (13) ein Bauteil ist, das innerhalb des Beatmungsgerätes (1) derart angeordnet ist, dass der Kanal (13) sich keine Wand mit dem Gehäuse (3) des Beatmungsgerätes (1) teilt.

6. Beatmungsgerät (1) nach einem der vorhergehenden Ansprüche, wobei der Spalt (10, 101) zwischen dem Gehäuse (3) und der Kammerwand (9) und/oder der Kanalwand (14) eine Größe von mindestens 0,5 mm, bevorzugt von mindestens 1 mm, besonders bevorzugt von mindestens 2 mm aufweist.

7. Beatmungsgerät (1) nach einem der vorhergehenden Ansprüche, wobei die Kammer (8) und/oder der Kanal (13) aus einem nicht-porösem Material ausgebildet sind, insbesondere in einen nicht porösen Kunststoff und/oder wobei die Kammer (8) und/oder der Kanal (13) ein Elastomer umfassen, wobei das Elastomer die Kammerwand (9) und/oder die Kanalwand (14) vorzugsweise derart umhüllt, dass Körperschwingungen der Kammer (8) und/oder des Kanals (13) gedämpft werden.

8. Beatmungsgerät (1) nach einem der vorhergehenden Ansprüche, wobei die Kanalwand (14) in den Innenraum (2) und/oder in die Kammer (8) hineinragend ausgebildet ist.

9. Beatmungsgerät (1) nach einem der vorhergehenden Ansprüche, wobei die Längsachsen von Saugstutzen (17) und Druckstutzen (18) dieselbe Orientierung aufweisen wie eine Längsachse des Kanals (13).

10. Beatmungsgerät (1) nach einem der vorhergehenden Ansprüche, wobei zwischen dem Atemgaseingang (4) und dem Atemgasausgang (5) ein Atemgasweg (6) zum Leiten des Atemgases ausgebildet ist, wobei der Atemgasweg (6) derart ausgebildet ist, dass das Atemgas mehrfach umgelenkt wird, wobei das Atemgas mindestens einmal U-förmig umgelenkt wird.

11. Beatmungsgerät (1) nach einem der vorhergehenden Ansprüche, wobei das Beatmungsgerät (1) mindestens ein Befestigungssystem (20) umfasst, das eingerichtet ist, um die Kammer (8) und/oder den Kanal (13) und/oder das Gebläse 7 mit dem Gehäuse (3) zu verbinden, wobei das Befestigungssystem (20) derart ausgebildet ist, dass zwischen dem Gehäuse (3) und der Kammer (8) ein Kontakt (23) ausgebildet ist, der möglichst gering ist, insbesondere unter 10%, bevorzugt unter 5%, besonders bevorzugt unter 2%.

12. Beatmungsgerät (1) nach Anspruch 11, wobei das Befestigungssystem (20) derart ausgebildet ist, dass zwischen dem Gehäuse (3) und dem Kanal (13) kein direkter Kontakt besteht und der Kanal (13) über eine Verbindung mit der Kammer (8) in dem Beatmungsgerät (1) gelagert ist.

13. Beatmungsgerät (1) nach einem der Ansprüche 11 oder 12, wobei das Befestigungssystem (20) als Mittelteil (20) ausgebildet ist und zumindest teilweise derart zwischen einem Unterteil des Gehäuses (3) und einem Oberteil des Gehäuses (3) positionierbar ist, so dass das Oberteil und das Unterteil atemgasdicht miteinander verbindbar sind.

14. Beatmungsgerät (1) nach einem der Ansprüche 11 bis 13, wobei das Mittelteil (20) eine Hartkomponente (39) und eine Weichkomponente (40) umfasst, wobei die Hartkomponente (39) zur Befestigung des Gebläses (7) und/oder der Kammer (8) und/oder des Kanals (13) ausgebildet ist, wobei die Weichkomponente (40) zur Abdichtung und/oder Vibrationsdämpfung ausgebildet ist und/oder wobei das Mittelteil (20) eine Durchführung (32) umfasst, die eingerichtet ist, um eine Passage für Leitungen und/oder Kabel aus dem Gehäuseinneren heraus zu ermöglichen, wobei die Durchführung (32) mindestens eine Durchführungsöffnung (37) umfasst, die von der Weichkomponente (40) umgeben ist.

15. Beatmungsgerät (1) nach einem der vorhergehenden Ansprüche, wobei das Beatmungsgerät (1) ein Gebläsehalterungssystem (24) mit einer Gebläseaufnahme (25) und einem Halteelement (26) umfasst, wobei das Halteelement (26) am Gebläse angeordnet ist und die Gebläseaufnahme (25) eingerichtet ist, um das Halteelement 26 zumindest teilweise derart aufzunehmen, so dass das Gebläse (7) innerhalb des Gehäuses (3) befestigt ist, wobei die Gebläseaufnahme (25) am Mittelteil (20) und/oder an der Kammerwand (9) und/oder an der Kanalwand (14) angeordnet ist oder durch diese ausgebildet ist.
